# EUROPEAN PATENT SPECIFICATION

(11) **EP 2 598 635 B1**
(45) Date of publication and mention of the grant of the patent: **22.10.2014**
(21) Application number: 11743572.7
(22) Date of filing: 29.07.2011
(51) Int. Cl.: C12N 5/079, C12N 5/0797, C12Q 1/02

(54) **CORTICOGENESIS OF HUMAN PLURIPOTENT CELLS**
KORTIKOGENESE VON MENSCHLICHEN PLURIPOTENTEN ZELLEN
CORTICOGÉNÈSE DE CELLULES PLURIPOTENTES HUMAINES

(30) Priority: 30.07.2010 US 400630 P
(43) Date of publication of application: 05.06.2013
(73) Proprietor: Cambridge Enterprise Limited, Trinity Lane, Cambridge CB2 1TN (GB)
(72) Inventor: LIVESEY, Frederick, John, Cambridge CB2 1QN (GB); SHI, Yichen, Cambridge CB2 1QN (GB)
(74) Representative: Sutcliffe, Nicholas Robert
(86) International application number: PCT/GB2011/001144
(87) International publication number: WO 2012/013936

(56) References cited:
- EP-A1- 2 128 244
- WO-A1-2007/142449
- HANSEN V ET AL: "Deriving excitatory neurons of the neocortex from pluripotent stem cells.", NEURON, vol. 70, no. 4, 26 May 2011 (2011-05-26), pages 645-660, XP002659753, ISSN: 1097-4199 cited in the application
- EIRAKU M ET AL: "Self-organized formation of polarized cortical tissues from ESCs and its active manipulation by extrinsic signals", CELL STEM CELL, vol. 3, no. 5, 6 November 2008 (2008-11-06), pages 519-532, XP008141935, ISSN: 1934-5909, DOI: 10.1016/J.STEM.2008.09.002 [retrieved on 2008-11-05] cited in the application
- GASPARD N ET AL: "An intrinsic mechanism of corticogenesis from embryonic stem cells", NATURE, vol. 455, no. 7211, 18 September 2008 (2008-09-18), pages 351-357, XP55007818, ISSN: 0028-0836, DOI: 10.1038/nature07287 cited in the application
- AU E & FISHELL G: "Cortex shatters the glass ceiling.", CELL STEM CELL, vol. 3, no. 5, 6 November 2008 (2008-11-06), pages 472-474, XP002659754, ISSN: 1875-9777 cited in the application
- LI X-J ET AL: "Coordination of sonic hedgehog and Wnt signaling determines ventral and dorsal telencephalic neuron types from human embryonic stem cells", DEVELOPMENT, vol. 136, no. 23, 1 December 2009 (2009-12-01), pages 4055-4063, XP55007823, ISSN: 0950-1991, DOI: 10.1242/dev.036624 cited in the application
- CHAMBERS S M ET AL: "Highly efficient neural conversion of human ES and iPS cells by dual inhibition of SMAD signaling", NATURE BIOTECHNOLOGY, vol. 27, no. 3, March 2009 (2009-03), pages 275-280, XP55007827, ISSN: 1087-0156, DOI: 10.1038/nbt.1529 cited in the application
- KIM D-S ET AL: "Robust Enhancement of Neural Differentiation from Human ES and iPS Cells Regardless of their Innate Difference in Differentiation Propensity", STEM CELL REVIEWS AND REPORTS, vol. 6, no. 2, June 2010 (2010-06), pages 270-281, XP55007831, ISSN: 1550-8943, DOI: 10.1007/s12015-010-9138-1
- SANSOM S N & LIVESEY F J: "Gradients in the brain: the control of the development of form and function in the cerebral cortex.", COLD SPRING HARBOR PERSPECTIVES IN BIOLOGY, vol. 1, no. 2, A002519, August 2009 (2009-08), XP002659755, ISSN: 1943-0264
- FAME R M ET AL: "Development, specification, and diversity of callosal projection neurons", TRENDS IN NEUROSCIENCE, vol. 34, no. 1, January 2011 (2011-01), pages 41-50, XP027581071, ISSN: 0166-2236 [retrieved on 2010-12-31] cited in the application
- PARK I-H ET AL: "Disease-specific induced pluripotent stem cells", CELL,, vol. 134, no. 5, 5 September 2008 (2008-09-05), pages 877-886, XP002552496, DOI: 10.1016/J.CELL.2008.07.041
- BEHRSTOCK S ET AL: "Human neural progenitors deliver glial cell line-derived neurotrophic factor to parkinsonian rodents and aged primates", GENE THERAPY, vol. 13, no. 5, March 2006 (2006-03), pages 379-388, XP55007837, ISSN: 0969-7128, DOI: 10.1038/sj.gt.3302679 cited in the application
- KLEIN S M ET AL: "GDNF Delivery Using Human Neural Progenitor Cells in a Rat Model of ALS", HUMAN GENE THERAPY, vol. 16, no. 4, April 2005 (2005-04), pages 509-521, XP55007838, ISSN: 1043-0342, DOI: 10.1089/hum.2005.16.509 cited in the application
- HANSEN D V ET AL: "Neurogenic radial glia in the outer subventricular zone of human neocortex", NATURE, vol. 464, no. 7288, 25 March 2010 (2010-03-25), pages 554-561, XP55007839, ISSN: 0028-0836, DOI: 10.1038/nature08845 cited in the application
- FIETZ S A ET AL: "OSVZ progenitors of human and ferret neocortex are epithelial-like and expand by integrin signaling", NATURE NEUROSCIENCE, vol. 13, no. 6, June 2010 (2010-06), pages 690-699, XP55007841, ISSN: 1097-6256, DOI: 10.1038/nn.2553 cited in the application

## Description

This invention relates to the induction of corticogenesis in pluripotent human cells *in vitro.*

The cerebral cortex is the integrative and executive centre of the mammalian central nervous system, making up over three quarters of the human brain (Mountcastle, V.B. The Cerebral Cortex, (Harvard University Press, Cambridge, Mass. 1998). Diseases of the cerebral cortex are major causes of morbidity and mortality in children and adults, ranging from developmental conditions such as epilepsy and autism to neurodegenerative conditions of later life, such as Alzheimer's disease. Much has been learned of the fundamental features of cerebral cortex development, function and disease from rodent models. However, the primate, and particularly the human cerebral cortex, differs in several respects from the rodent (Finlay, B.L. et al. Science 268, 1578-84 (1995)). In addition to a marked increase in the size of the cerebral cortex relative to the rest of the nervous system, these include the size, complexity, and the nature of its developing stem cell populations (Rakic, P. Nat Rev Neurosci 10, 724-35 (2009)), an increase in the diversity of upper layer, later born neuronal cell types and the presence of primate specific neuron types in deep layers (Hill, R.S. et al Nature 437, 64-7 (2005)). Methods to model human cortical development in a controlled, defined manner from embryonic and induced pluripotent stem cells (collectively referred to as pluripotent stem cells, PSCs) have considerable potential to enable functional studies of human cortical development, circuit formation and function, and for constructing in vitro models of cortical diseases. Given that many of the major diseases of the cerebral cortex are diseases of synaptic function, a goal of the field is to generate cortical networks in vitro that closely resemble those found in vivo.

The cerebral cortex contains two major classes of neurons: approximately 80% are excitatory, glutamatergic projections neurons, generated by cortical stem and progenitor cells, whereas the remaining 20% are GABAergic interneurons that are generated outside the cortex and migrate in during development (Wonders, C.P et al. Nat Rev Neurosci 7, 687-96 (2006)). Glutamatergic projection neurons destined for the six layers of the adult cortex are generated in a stereotyped temporal order, with deep layer neurons produced first and upper layer neurons last. In mice, this process takes approximately six days, whereas in humans cortical neurogenesis lasts for over 70 days (Caviness, V.S., Jr et al Trends in Neurosciences 18, 379-83 (1995)). Once generated, the different classes of cortical projection neurons form canonical local microcircuits between cortical layers (Douglas, R.J. et al. Annu Rev Neurosci 27, 419-51 (2004)), as well as longer-range intra- and extra-cortical connections, including corticospinal tract, corticothalamic and callosal projections (Fame, R.M., et al Trends in neurosciences 34, 41-50 (2011); Lopez-Bendito, G et al Nature reviews. Neuroscience 4, 276-89 (2003)).

Although mouse ES cells have been shown to be competent to differentiate to cerebral cortex neurons in *vitro* by inhibition of sonic hedgehog signalling during neural induction (Bibel, M. et al. Nat Neurosci 7, 1003-9 (2004); Gaspard, N. et al. Nature 455, 351-7 (2008); Eiraku, M. et al. Cell Stem Cell 3, 519-32 (2008)), a common problem for efforts to model cortical development is that whereas production of deep layer, early-born neurons has been achieved, the complete programme of cortical neurogenesis has not been executed from pluripotent stem cells in culture. This is particularly the case for human corticogenesis from ES cells, which to date has not been achieved in a defined, robust and efficient manner (Au, E. et al. Cell stem cell 3 472-4 (2008)). It has been proposed that one reason for this failure is that directed differentiation from ES cells does not reproduce the complex stem/progenitor cell populations found in the cortex in vivo (Hansen, D.V. et al. Neuron 70, 645-60 (2011)).

While neuroepithelial ventricular zone cells are the primary stem/progenitor population of the cerebral cortex, at least two secondary progenitor populations, basal progenitors/subventricular zone cells and outer subventricular zone (oSVZ) cells have been identified in mouse, ferret and humans. All three groups of stem/progenitor cells appear to generate projection neurons (Hansen, D.V., et al. Nature 464, 554-561 (2010); Wang, X., et al. Nature neuroscience 14, 555-61 (2011); Fietz, S.A. et al. Nat Neurosci 13, 690-9 (2010)), and the increased numbers of oSVZ cells in the primate, and particularly the human, cerebral cortex has been proposed to be a key contributor to the increased size of the human cortex, as well as the diversification of upper layer neuron types.

Although has been shown previously that some classes of cortical neurons are generated in aggregate cultures of human ES cells (US20100166720A1; Eiraku, M., et al. Cell stem cell 3, 519-532 (2008); Li, X.J., et al. Development 136, 4055-4063 (2009)), the inhibition of sonic hedgehog does not appear to function for corticogenesis from human pluripotent stem cells in monolayer culture. Neural stem and progenitor cells have been differentiated from human ES cells using embryoid body intermediates (WO2007142449).

This invention relates to the development of a process to induce human pluripotent cells to undergo corticogenesis at high efficiency *in vitro*. This may be useful, for example, in production of cortical neurons, in particular patient-specific cortical neurons; the modelling of juvenile and adult-onset neurological diseases; and the development of therapeutics to these diseases.

An aspect of invention provides a method for *in vitro* induction of corticogenesis of human pluripotent cells comprising;
(i) providing a population of isolated human pluripotent stem cells,
(ii) culturing the population under culture conditions which stimulate retinoid signalling and inhibit TGFβ and BMP signalling,
such that said population differentiate into cortical stem and progenitor cells.

Cortical stem and progenitor cells may be maintained in culture, stored, for example frozen using conventional techniques, or used in therapeutic or other applications as described herein.

In some embodiments, the cortical stem and progenitor cells may be differentiated into cortical neurons. A method may further comprise;
(iii) allowing the population of cortical stem and progenitor cells to differentiate into cerebral cortex neurons.

Human stem pluripotent cells are unspecialized, undifferentiated cells that are capable of replicating or self-renewing themselves and developing into specialized cells of all three primary germ layers i.e. ectoderm, mesoderm and endoderm but are not able to develop into all embryonic and extra-embryonic tissues, including trophectoderm (i.e. not totipotent). The human stem pluripotent cells are not committed to a neural lineage.

Human pluripotent cells include embryonic stem (ES) cells and non-embryonic stem cells, including foetal and adult somatic stem cells and stem cells derived from non-pluripotent cells.

In other embodiments, the human pluripotent cells may be induced pluripotent (iPS) cells which are derived from non-pluripotent cells. iPS cells are described in more detail below.

A human pluripotent stem cell may express one or more of the following pluripotency associated markers: Oct4, Sox2, alkaline phosphatase, SSEA-3, Nanog, SSEA-4 and Tra-1-60. Preferably, human pluripotent stem cells express Oct4.

Human pluripotent stem cells do not express neural cell markers, such as Tuj1.

Markers expressed by a cell, including pluripotency associated markers, may be identified using standard techniques, such as PCR, western blotting, immunocytochemistry and *in situ* hybridisation.

A population of human pluripotent cells for use in the present methods may be obtained by culturing cells from a pluripotent cell line, using conventional techniques (Vallier, L. et al Dev. Biol. 275, 403-421 (2004), Cowan, C.A. et al. N. Engl. J. Med. 350, 1353-1356 (2004), Joannides, A. et al. Stem Cells 24, 230-235 (2006) Klimanskaya, I. et al. Lancet 365, 1636-1641 (2005), Ludwig, T.E. et al. Nat. Biotechnol. 24, 185-187 (2006)). For example, human pluripotent cells suitable for use in the present methods may be conventionally cultured in a culture dish on a layer of feeder cells, such as irradiated mouse embryonic fibroblasts (MEF), at an appropriate density (e.g.10⁵ to 10⁶ cells/60mm dish), or on an appropriate substrate with feeder conditioned or defined medium. Human pluripotent cells for use in the present methods may be passaged by enzymatic or mechanical means. Suitable culture media for human pluripotent cells include SC medium (Knockout Dulbecco's Modified Eagle's Medium (KO-DMEM) supplemented with 20% Serum Replacement, 1% Non-Essential Amino Acids, 1mM L-Glutamine, 0.1mM β-mercaptoethanol and 4ng/ml to 10ng/ml human bFGF) and ES medium (DMEM/F12 supplemented with 20% knockout serum replacement (KSR), 6 ng/ml FGF2 (PeproTech), 1mM L-Gln, 100 µm non-essential amino acids, 100 µM 2-mercaptoethanol, 50 U/ml Penicillin and 50 mg/ml Streptomycin).

A population of human pluripotent stem cells for induction of *in vitro* corticogenesis is preferably substantially free from one or more other cell types. As described above, human pluripotent cells are typically cultured and maintained on MEF feeder cells. Human pluripotent cells may be separated from the feeder cells by any suitable technique. For example, the cells may be briefly (e.g. one hour) cultured on gelatin, and then the human pluripotent cells, which do not adhere to the gelatin separated from the MEFs which do adhere to the gelatin.

This avoids the formation of embryoid bodies, as discussed below.

Following separation, human pluripotent cells may be cultured in a monolayer on suitable medium, for example the SC or ES media described above, supplemented with 10 ng/ml FGF2. Preferably, the medium contains a Rho-associated, coiled-coil containing protein kinase (ROCK) inhibitor (e.g. 10 µM), to reduce cell death when the human pluripotent cells are dissociated into single cell suspension (Olson, M.F. (2008). Curr Opin Cell Biol 20: 242-8; Watanabe, K.et al. (2007) Nat Biotechnol 25: 681-6, US 2010/0009442). Suitable ROCK inhibitors are well knownin the art and include include (+)-4-[1(R)-aminoethyl]-N-(1H-pyrrolo[2,3-b]pyridine-4-yl)benzamide dihydrochloride hydrate; (1R,4R)-4-((R)-1-aminoethyl)-N-(pyridine-4-yl)cyclohexanecarboxamide dihydrochloride; and N-(2-(2-(dimethylamino)ethoxy)-4-(1H-pyrazol-4-yl)phenyl)-2,3-dihydrobenzo[b][1,4]dioxine-2-carboxamide (all available from Stemgent USA or Calbiochem USA).

A monolayer is a single layer of cells on a substrate, such as the surface of a culture plate. Monolayer culture of the human pluripotent cells allows controlled, defined differentiation and prevents the formation of embryoid bodies. Embryoid bodies are aggregates formed by the uncontrolled differentiation of stem cells which consist of various types of differentiated cell and from which neural stem cells have to be further purified. Monolayers may also allow the long-term imaging of cultures.

Preferably, human pluripotent stem cells are cultured and differentiated in monolayer culture as described herein without the formation of embryoid bodies.

Before initiation of *in vitro* corticogenesis, the population of isolated human pluripotent stem cells may be expanded. For example, the human pluripotent stem cells may be cultured in a monolayer under conditions that simulate FGF2 signalling. In some embodiments, the cells may be cultured in a culture medium supplemented with FGF2 (e.g. 5 to 20 ng/ml FGF2, preferably 10ng/ml). Suitable culture media include the SC and ES media described above, which may be MEF-conditioned and supplemented with FGF2.

Any mammalian FGF2 may be employed, preferably human fibroblast growth factor 2(FGF2) (NCBI GeneID: 2247, nucleic acid sequence NM_002006.3 GI: 41352694, amino acid sequence NP_001997.4 GI: 41352695). FGF2 may be produced using routine recombinant techniques or obtained from commercial suppliers (e.g. R&D, Minneapolis, MN, USA).

Preferably, the population of human pluripotent stem cells for initiation of *in vitro* corticogenesis is provided in monolayer culture. Methods for the monolayer culture of human pluripotent cells are well known in the art.

Preferably, corticogenesis is induced when the human pluripotent cells in the monolayer culture are at least 85%, at least 90%, or at least 95% confluent (i.e. at least 85% of the surface of the culture vessel is covered by the cells).

As described above, corticogenesis is initiated by culturing the human pluripotent stem cells under culture conditions which stimulate retinoid signalling and inhibit TGFβ superfamily signalling.

TGFβ superfamily signalling is mediated by SMAD proteins (for example SMAD1-3, 5 and 9) and may be inhibited by inhibiting TGFβ and BMP signalling in the human pluripotent cells (Chambers, S.M., et al Nat Biotechnol 27, 275-280(2009), Schmierer et al Nat Rev Mol Cell Biol. 2007 Dec;8(12):970-82). TGFβ-SMAD signalling may be inhibited by TGFβ and BMP signalling inhibitors in the neural induction medium.

Preferably, once neural induction is initiated, retinoid signalling stimulation and TGFβ and BMP signalling inhibition are constantly maintained until the population of human pluripotent cells differentiate into cortical stem and stem and progenitor cells.

For example, the cells may be cultured in a neural induction medium comprising one or more factors which stimulate or promote retinoid signalling and inhibit TGFβ and BMP signalling in the cells.

The neural induction medium may comprise a retinoid which stimulates retinoid signalling in the human pluripotent stem cells. Suitable retinoids are well-known in the art and include retinoic acid, vitamin A (all trans retinol), and retinol acetate. For example, the neural induction medium may comprise 0.01 µM to 10 µM all-trans retinol, typically 0.5 µM.

Techniques for the stimulation of retinoid signalling in pluripotent cells are well-known in the art (see for example WO2008071960A2, US20070224650A1 US7632679B2, WO2008060792A2, WO2009058451A2 US20060073587, and WO2005021720).

TGFβ superfamily signalling may be inhibited by one or more TGFβ-SMAD signalling inhibitors in the neural induction medium. TGFβ-SMAD signalling inhibitors may include TGFβ signalling inhibitors and BMP signalling inhibitors.

The neural induction medium may comprise a TGFβ signalling inhibitor. TGFβ signalling occurs through the SMAD2 and SMAD3 mediated pathway and may be mediated by TGF-β1 activin receptor-like kinases (ALKs) ALK-4, -5 and -7 (Schmierer et al Nat Rev Mol Cell Biol. 2007 Dec;8(12):970-82). A TGFβ signalling inhibitor may inhibit SMAD2 and SMAD3 mediated signalling.

Suitable TGFβ signalling inhibitors include inhibitors of ALK 4, 5 and 7 receptors, such as 2-(5-benzo[1,3]dioxol-5-yl-2-*tert*-butyl-3H-imidazol-4-yl)-6-methylpyridine hydrochloride (SB-505124); 4-[4-(1,3-benzodioxol-5-yl)-5-(2-pyridinyl)-1H-imidazol -2-yl] benzamide (SB431542; Tocris Bioscience USA; Stemgent USA), and 3-(6-methyl-2-pyridinyl)-N-phenyl-4-(4-quinolinyl)-1H-pyrazole-1-carbothioamide (A83-01; Tocris Bioscience USA; Stemgent USA).

SB431542 is an inhibitor of the TGF-β1 activin receptor-like kinases (ALKs). It is a selective and potent inhibitor of ALK-4, -5 and - 7, and thus blocks BMP-mediated SMAD 2/3 phosphorylation (Laping, N. J. et al., Mol. Pharmacol., 62:58-64 (2002)).

For example, the neural induction medium may comprise 5 µM to 20 µM SB431542, for example about 10 µM.

Methods for the inhibition of TGFβ signalling in human pluripotent cells are well-known in the art (see for example US7250294, US7560281 and WO20100638481; Chambers, S.M., et al Nat Biotechnol 27, 275-280(2009)).

The neural induction medium may comprise a BMP signalling inhibitor. BMP signalling occurs through the SMAD1, SMAD5 and SMAD8 mediated pathway and may be mediated by TGF-β1 activin receptor-like kinases (ALKs) -1, -2 and -3 and -6 (Schmierer et al Nat Rev Mol Cell Biol. 2007 Dec;8(12):970-82). A BMP signalling inhibitor may inhibit SMAD1, SMAD5 and SMADB mediated signalling.

Suitable BMP signalling inhibitors may inhibit signalling through the SMAD1, SMAD5 and SMAD8 (also called SMAD9) mediated pathway in the human pluripotent stem cells. BMP signalling is mediated by BMP type I receptors ALK1, ALK2, ALK3 and ALK6 and suitable BMP signalling inhibitors include inhibitors of ALK1, ALK2, ALK3 and ALK6 receptors

Suitable BMP signalling inhibitors are known in the art (Cuny et al (2008) Bioorg Med Chem Lett 18 4388-4392; Yu et al (2008) Nat Med 14 1363-9) and include noggin, dorsomorphin, follistatin, inhibin, sclerostin, chordin, CTGF, gremlin and 4-(6-(4-(piperazin-1-yl)phenyl)pyrazolo[1,5-a]pyrimidin-3-yl)quinoline (LDN-193189 Stemgent USA).

In some embodiments, the BMP inhibitor may be noggin. Noggin is a secreted homodimeric glycoprotein that binds and inactivates members of the transforming growth factor-beta (TGF-β) superfamily of signaling proteins, such as bone morphogenetic protein-4 (BMP4) (Groppe et al Nature 420, 636-642).

Any mammalian Noggin may be used. For example, human noggin (NOG) (NCBI GeneID: 9241, nucleic acid sequence NM_005450.4 GI: 189339247, amino acid sequence NP_005441.1 GI: 4885523) or mouse noggin (Nog) (NCBI 18121, nucleic acid sequence NM_008711.2 GI: 158187522 amino acid sequence NP_032737.1 GI: 7110675) may be produced using routine recombinant techniques or obtained from commercial suppliers (e.g. R&D Systems, Minneapolis, MN, USA).

For example, the neural induction medium may comprise 250 ng/ml to 1000 ng/ml, for example about 500 ng/ml noggin.

In some embodiments, noggin may be linked to other moieties. For example, a chimeric noggin molecule, such as a mouse noggin-Fc chimera (R&D systems) may be employed.

In other embodiments, the BMP inhibitor may be dorsomorphin (6-[4-[2-(1-Piperidinyl)ethoxy]phenyl]-3-(4-pyridinyl)-pyrazolo[1,5-a]pyrimidine dihydrochloride). Dorsomorphin functions through inhibition of BMP type I receptors ALK2, ALK3 and ALK6 and thus blocks BMP-mediated SMAD1/5/8 phosphorylation. Dorsomorphin inhibits BMP signals required for embryogenesis and iron metabolism (Yu et al., Nat Chem Biol 4: 33-41). Dorsomorphin may be obtained from commercial suppliers (e.g. Tocris Bioscience USA; Stemgent USA).

Methods for the inhibition of BMP signalling in pluripotent cells are well-known in the art (see for example WO2008026198A2; Chambers, S.M., et al Nat Biotechnol 27, 275-280(2009)).

In some preferred embodiments, the one or more TGFβ-SMAD signalling inhibitors in the neural induction medium inhibit both SMAD2/3 mediated signalling and SMAD1/5/8 mediated signalling. For example, the neural induction medium may comprise a TGFβ signalling inhibitor, such as SB431542, and a BMP signalling inhibitor, such as noggin, as described above.

The neural induction medium may further comprise insulin. This may, for example, improve the survival rates of the cortical stem and progenitors and neurons. For example, the neural induction medium may comprise 1 µg/ml to 10 µg/ml insulin, for example about 5 µg/ml.

As described above, preferably, the human pluripotent cells are cultured under conditions which inhibit signalling of TGFβ superfamily, which includes both TGFβ and BMP. This may be achieved by inhibition of both TGFβ and BMP signalling pathways and results in the inhibition of all SMAD signalling in the human pluripotent cells, for example signalling mediated by SMAD 1 to 3, 5 and 9.

A suitable neural induction medium may therefore comprise a retinoid, a TGFβ signalling inhibitor, a BMP inhibitor and insulin, as described above.

The neural induction medium may also comprise standard neural cell culture reagents. For example, the medium may comprise a basal neural culture medium, such as DMEM/F12 (GIBCO) supplemented with N2 (Bottenstein et al 1979 PNAS USA 76 1 514-517; GIBCO), or Neurobasal (Invitrogen) supplemented with B27 (GIBCO/Invitrogen).

Other suitable basal neural culture media and supplements are well known in the art and/or available from commercial sources. For example, NS21 supplement (Chen et al Journal of Neuroscience Methods 171 (2008) 239-247) may be used instead of B27.

In some embodiments, one or more of the retinoid, TGFβ signalling inhibitor, BMP inhibitor and insulin may be supplied as part of a standard medium. For example, retinoic acid may be supplied as a component of the B27 medium supplement.

The basal neural medium may be supplemented with antibiotics such as streptomycin and penicillin, non-essential amino acids, L-glutamine, and reducing agents such as mercaptoethanol, as required.

A suitable neural induction medium may be based on a standard medium which supports neural induction, neurogenesis and neuronal differentiation. A 3N medium may comprise a 1:1 mixture of N2-containing and B27 media-containing, wherein the N2-containing medium comprises MEM/F12 supplemented with N2 (GIBCO), insulin, L-glutamine, non-essential amino acids, 2-mercaptoethanol, penicillin and Streptomycin and the B27-containing medium comprises neurobasal (Invitrogen) supplemented with B27 supplement(GIBCO), L-glutamine, penicillin and streptomycin.

Typically, N2 medium may comprise 5 µg/ml Insulin, 1mM L-Glutamine, 100 µm non-essential amino acids, 100 µM 2-mercaptoethanol, 50 U/ml Penicillin and 50 mg/ml Streptomycin and B27 medium may comprise 200 mM Glutamine, 50 U/ml Penicillin and 50 mg/ml Streptomycin.

The 3N medium described above may be supplemented with TGFβ and BMP signalling inhibitors to produce a neural induction medium.

The culture of mammalian cells is well-known in the art (see, for example, Basic Cell Culture Protocols, C. Helgason, Humana Press Inc. U.S. (15 Oct 2004) ISBN: 1588295451; Human Cell Culture Protocols (Methods in Molecular Medicine S.) Humana Press Inc., U.S. (9 Dec 2004) ISBN: 1588292223; Culture of Animal Cells: A Manual of Basic Technique, R. Freshney, John Wiley & Sons Inc (2 Aug 2005) ISBN: 0471453293, Ho WY et al J Immunol Methods. (2006) 310:40-52, Handbook of Stem Cells (ed. R. Lanza) ISBN: 0124366430). Media and ingredients thereof may be obtained from commercial sources (e.g. Gibco, Roche, Sigma, Europabioproducts, R&D Systems).

Standard mammalian cell culture conditions may be employed, for example 37°C, 21% Oxygen, 5% Carbon Dioxide. Media is preferably changed every two days and cells allowed to settle by gravity. Coveniently, cells are cultured on a surface coated with extracellular matrix components, such as Matrigel^{™}.

The human pluripotent stem cells may be cultured in the neural induction medium for 5 or more, 10 or more, or 15 or more days. For example the cells may be cultured for up to 15, up to 20 days or to 25 days, typically 8 to 11 days, to allow the conversion of human pluripotent cells into cortical stem and stem and progenitor cells.

Culturing human pluripotent cells in the neural induction medium as described above induces the cells to differentiate into cortical stem and progenitor cells. For example, following culture in the neural induction medium, 85% or more, 90% or more, 95% or more or 98% or more of the human pluripotent stem cells in the population may have differentiated into cortical stem and progenitor cells.

Preferably, 95% or more of the human pluripotent stem cells in the population may have differentiated into cortical stem and progenitor cells within 14 days of the initiation of differentiation.

Cortical stem and progenitor cells are daughter or descendant of a undifferentiated human pluripotent stem cell and has a committed cortical phenotype and reduced differentiation potential compared to the original stem cell. Cortical stem and progenitor cells, for example, are able to further differentiate into cerebral cortical neurons of any class or laminar fate, for example neurons of any one of layers 1 to 6 of the cerebral cortex.

The population of cortical stem and progenitor cells produced by neural induction of human pluripotent cells as described herein includes both cortical cells that can be propagated and remain multipotent (cortical stem cells) and cells with more restricted potential that are not necessarily able to self-renew (progenitor cells).

Cortical stem and progenitor cells may form neuroepithelial rosettes in culture. These neuroepithelial rosettes may display one or more features of the cortical neuroepithelium *in vivo*, such as; apico-basal polarity; apical mitoses, and significant amounts of abventricular mitoses. The population of cortical stem and progenitor cells may comprise apical and basal cortical stem and progenitor cells.

Cortical stem and progenitor cells may express Pax6. Cortical stem and progenitor cells may also express one or more of FoxG1, Emx1, Emx2 and COUP-TF1.

A subset of the population of cortical stem and progenitor cells (i.e. basal cells) may also express Tbr2.

Cortical stem and progenitor cells do not express pluripotency associated markers, such as Oct4, Sox2, Alkaline Phosphatase, SSEA-3, Nanog, SSEA-4 and Tra-1-60.

A method may comprise monitoring or detecting the expression of one or more cortical stem and progenitor cell markers and/or one or more pluripotent cell markers in cells in the population. This allows the extent of differentiation or neural induction of the population to be determined as it is cultured in the neural induction medium.

Cortical stem and progenitor cells produced by the present methods may be substantially free from other cell types. For example, the population of cells may contain 80% or more, 85% or more, 90% or more, or 95% or more cortical stem and progenitor cells, following culture in the neural induction medium.

Preferably, the population of cortical stem and progenitor cells is sufficiently free of other cell types that no purification is required.

Following culturing in the neural induction medium as described above, the population of cortical stem and progenitor cells may be isolated and/or removed from the neural induction medium. Suitable techniques are well known in the art.

In some embodiments, the population of cortical stem and progenitor cells may be expanded. Suitable culture conditions for expansion of cortical stem and progenitor cells include conditions that simulate FGF2 signalling. For example, the population of cortical stem and progenitor cells may be expanded by culturing in an expansion medium supplemented with fibroblast growth factor 2 (FGF2). For example, a medium comprising 10 to 40 ng/ml FGF2, preferably 20ng/ml

Suitable expansion media may include the 3N medium described above, supplemented with FGF2. Other suitable media would be apparent to the skilled person.

Following the production and optional expansion of cortical stem and progenitor cells, neurogenesis of the cortical stem and progenitor cells may be initiated to produce a population of cerebral cortex neurons.

The population of cortical stem and progenitor cells may be cultured in conditions which promote neurogenesis. For example, the cells may be cultured an expansion medium, such as 3N medium as described above, without FGF2.

The population of cortical stem and progenitor cells may be cultured for at least 40, at least 60, at least 80, at least 100, or more than 100 days, or until neurogenesis is complete or a sufficient amount of neurogenesis has occurred. Standard cell culture techniques may be employed.

Cerebral cortex neurons are fully differentiated functional glutamatergic projection neurons. Cerebral cortex neurons may express one or more markers selected from the group consisting of Tbr1, CTIP2, Cux1, Satb2, Brn2, reelin, Fezf1, Fezf2, Sox5, Bhlhb5, Pou3f1 and Otx1 (see Molyneaux et al., Nat Rev Neurosci. 2007 Jun;8(6):427-37).

A method may comprise monitoring or detecting the expression of one or more cerebral cortex neuronal markers and/or one or more cortical stem and progenitor cell markers in cells in the population.

The cerebral cortex neurons may be of any class. For example, the neurons may be cortical layer 6, cortical layer 5, cortical layer 4, cortical layer 3, cortical layer 2, or cortical layer 1 neurons.

Cerebral cortex neurons of different classes may be identified by the expression of neuronal class markers. For example, layer 1 neurons express reelin; layers 2/3 neurons express Brn2; layers 2-4 neurons express Cux1 & Satb2; layer 5 neurons express CTIP2, Pou3f1/SCIP or Otx1; layer 5 corticospinal motor neurons express CTIP2 and not Tbr1; and layer 6 neurons express Tbr1 and Fezf2.

The expression of neuronal class markers may be determined by any suitable technique, including immunocytochemistry, immunofluoresence and RT-PCR.

Different classes of cerebral cortical neurons may be generated progressively following initiation of differentiation of the cortical stem and progenitor cells, for example over 80, 90 or 100 days. In some embodiments, deep, layer 6 neurons differentiate before layer 5 corticospinal motor neurons, with superficial layer (layers 2-4) neurons appearing subsequently. Roughly equal amounts of deep and superficial layer neurons may be produced.

Cells may be cultured until the desired class of cerebral cortical neurons is produced. The classes of neurons which are present in the cell culture may be monitored by detecting the expression of neuronal class markers, as described above.

In some embodiments, the rate of neurogenesis may be controlled for example by adding or withdrawing FGF2 from the culture medium, to slow down the appearance of a particular neuronal cell class. A method may comprise isolating a population of neurons of a particular class from the cell culture. For example, a population of cortical layer 6, cortical layer 5, cortical layer 4, cortical layer 3, cortical layer 2 or cortical layer 1 neurons may be isolated. These neurons may be useful in a range of therapeutic and other applications as described below.

In some preferred embodiments, a population of cortical layer 5 neurons may be isolated. These include corticospinal motor neurons which may be especially useful in treating or modelling spinal cord injury and motor neuron disease or screening for therapeutics for these conditions.

Cortical neurons produced by the present methods, in particular cortical neurons of a particular class or subset, such as corticospinal motor neurons, may be substantially free from other cell types. In some embodiments, cortical neurons of interest may be separated from other cell types and classes in the cell culture using any technique known to those skilled in the art, including those based on the recognition of extracellular epitopes such as neuronal class markers by antibodies, or magnetic beads or fluorescence activated cell sorting (FACS).

Cerebral cortex neurons produced as described may display functional electrophysiological properties and form neuronal synapses. The excitatory synaptic properties of a population of cerebral cortex neurons may be determined, for example by detecting the presence of miniature excitatory postsynaptic potentials (mEPSPs) or the presence of foci of synaptophysin immunofluorescence. This may be done using standard techniques.

As described above, the human pluripotent stem cells may be induced pluripotent stem (iPS) cells.

iPS cells are pluripotent cells which are derived from non-pluripotent, fully differentiated ancestor cells. Suitable cells include adult fibroblasts and peripheral blood cells. Ancestor cells are typically reprogrammed by the introduction of pluripotency genes or proteins, such as Oct4, Sox2 and Sox1 into the cell. The genes or proteins may be introduced into the differentiated cells by any suitable technique, including viral or plasmid transfection or direct protein delivery. Other genes, for example Kif genes, such as Kif-1, -2, -4 and -5; Myc genes such as C-myc, L-myc and N-myc; nanog; and Lin28 may also be introduced into the cell to increase induction efficiency. Following introduction of the pluripotency genes or proteins, the ancestor cells may be cultured. Cells expressing pluripotency markers may be isolated and/or purified to produce a population of iPS cells. Techniques for the production of iPS cells are well known in the art. (Yamanaka et al Nature 2007; 448:313-7; Yamanaka 6 2007 Jun 7;1(1):39-49. Kim et al Nature. 2008 Jul 31; 454(7204):646-50; Takahashi Cell. 2007 Nov 30; 131(5):861-72. Park et al Nature. 2008 Jan 10;451(7175):141-6; Kimet al Cell Stem Cell. 2009 Jun 5;4(6):472-6.)

In some embodiments, iPS cells may be derived from healthy cells obtained from an individual, i.e. cells without a disease-associated phenotype or genotype. In some embodiments, cells may be obtained from a patient with damaged or dysfunctional cortical neurons, for example an individual with a neurological disease, head trauma, multiple sclerosis, stroke or spinal cord injury. Cortical neurons produced from these cells may be useful in treating the patient.

In some embodiments, iPS cells may be disease-specific iPS cells. Disease-specific iPS cells may be derived from disease associated cells from an individual i.e. cells with a phenotype or genotype associated with disease, for example a neurological disease, such as sporadic and familial Alzheimer's disease, familial and sporadic epilepsy, autism, schizophrenia and cerebral palsy.

Disease associated cells for the production of iPS cells may be obtained from an individual suffering from a neurological disease or susceptible to or at risk of a neurological disease.

Neurological diseases include diseases associated with damaged or dysfunctional cortical neurons, such as sporadic and familial Alzheimer's disease, familial and sporadic epilepsy, autism, schizophrenia and cerebral palsy.

In some preferred embodiments, the neurological disease is a juvenile or adult onset neurodegenerative disease.

Disease-specific iPS cells may be differentiated as described herein to produce populations of cortical neurons which are useful as models of the neurological disease. In particular, cortical neurons produced from disease specific iPS cells as described herein may display disease pathologies over a short time frame (i.e. weeks or months). This facilitates the screening of therapeutic molecules.

In some preferred embodiments, the human pluripotent stem cells are Down syndrome iPS cells (DS-iPS cells). DS-iPS cells are iPS cells which are derived from cells obtained from individuals with Down syndrome (Park, I.H., et al Cell 134, 877-886 (2008)).

Down syndrome/Trisomy 21 is the commonest genetic cause of mental retardation in humans (Wiseman, F.K et al Hum Mol Genet 18, R75-83 (2009)). Individuals with Down syndrome have a very high incidence of Alzheimer's disease, attributed to the presence of the amyloid precursor protein (APP) gene on chromosome 21 (Rumble, B., et al. N Engl J Med 320, 1446-1452 (1989); Selkoe, D. J.Biol Chem 271, 18295-18298 (1996)). *In vitro* induction of corticogenesis in DS-iPS cells as described herein leads to the production of cerebral cortex neurons with an extra copy of chromosome 21.

These cerebral cortex neurons display AD pathology in cell culture. For example, DS-iPS derived cortical neurons produce high levels of the Aβ42 fragment of amyloid precursor protein (APP), form intra- and extra-cellular amyloid plaques, and display increased rates of programmed cell death.

A method of producing cortical neurons with AD pathology may comprise;
inducing *in vitro* corticogenesis of a population of DS-iPS cells as described above,
thereby producing a population of cortical neurons with AD pathology.

DS-iPS derived cortical neurons may display AD pathologies within 1, 2, 3, 4 or more months of the initiation of differentiation from the DS-iPS cell or stored cortical stem cells derived therefrom.

Once produced, cortical neurons with AD pathology may be cultured and maintained, for example for use in screening. A method of maintaining cortical neurons with AD pathology may comprise;
culturing a population of cortical neurons derived from DS-iPS cells, as described above.

Methods of culturing neurons are well known in the art.

A method may further comprise measuring or detecting one or more AD pathologies in said cells.

AD pathologies include increased expression levels of Aβ42, increased ratio of Aβ42 to Aβ40 (non-toxic form); increased Aβ42 levels within neurons; increased Ab42 levels in the extracellular medium; increased Aβ42 oligomer formation; increased levels of hyperphosphorylated Tau; increased intracellular calcium levels; increased formation of intra- and extracellular amyloid plaques; and increased rates of programmed cell death.

This may be useful, for example, in screening methods, which are described in more detail below.

Individuals with Down syndrome may display premature aging. *In vitro* induction of corticogenesis in DS-iPS cells as described herein leads to the production of cerebral cortex neurons which may be useful in the study of aging and age-related cognitive decline.

A method of producing cortical neurons with age-related pathology may comprise;
inducing *in vitro* corticogenesis of a population of DS-iPS cells as described above,
thereby producing a population of cortical neurons with age-related pathology.

DS-iPS derived cortical neurons may display age-related pathology within 1, 2, 3, 4 or more months of the initiation of differentiation from the DS-iPS cell or stored cortical stem cells derived therefrom.

Once produced, cortical neurons with age-related pathology may be cultured and maintained, for example for use in screening. A method of maintaining cortical neurons with age-related pathology may comprise;
culturing a population of cortical neurons derived from DS-iPS cells, as described above.

Age related pathologies may include reduced growth, increased rates of programmed cell death, reduced synaptic activity and reduced excitatory activity.

An isolated cerebral cortex neuron or an isolated cortical stem and progenitor cell may be produced by the process of *in vitro* corticogenesis as described above. A population of isolated cerebral cortex neurons or isolated cortical stem and progenitor cells may be produced by the process of *in vitro* corticogenesis as described above.

A population of isolated cerebral cortex neurons may comprise 80% or more, 85% or more, 90% or more, 95% or more, or 99% or more glutamatergic projection neurons with functional excitatory properties. The population may comprise less than 1% interneurons, preferably no interneurons, or no interneurons detectable by immunocytology.

A population of isolated cortical stem and progenitor cells may comprise 80% or more, 85% or more, 90% or more, 95% or more, or 99% or more cortical stem and progenitor cells.

Cortical stem and progenitor cells produced by the methods described herein are dorsal telencephalic cells (i.e. they are specified to cortex tissue).

Populations of cortical stem and progenitor cells produced by the methods described herein do not contain hindbrain or spinal cord stem and progenitor cells.

The neurons or stem and progenitor cells may be produced from iPS cells derived from an individual with a dysfunctional (e.g. damaged or diseased) cerebral cortex. These cortical stem and progenitor cells or neurons may be used in the treatment of the patient. For example, cortical cells may be administered to replace damaged cortical neurons in an individual with a dysfunctional cerebral cortex i.e. an individual with diseased or dysfunctional cortical neurons.

A population of cortical stem and progenitor cells or cortical neurons produced by a method described herein may be used in a method of treatment of the human or animal body, for example in the treatment of a patient with a dysfunctional cerebral cortex and the population of cortical stem and progenitor cells or cortical neurons produced by a method described herein may be used in the manufacture of a medicament for use in the treatment of dysfunctional cerebral cortex.

An individual with a dysfunctional cerebral cortex may have disease associated with damaged or dysfunctional cortical neurons, such as sporadic and familial Alzheimer's disease, familial and sporadic epilepsy, autism, schizophrenia, motor neurone disease, cerebral palsy, multiple sclerosis, or stroke or may have an suffered injury or trauma to the brain or spinal cord. For example, corticospinal motor neurons (layer 5) produced as described above may be used to treat spinal cord injury.

Preferably, the population of cortical stem and progenitor cells or cortical neurons used to treat an individual are produced from iPS cells derived from cells obtained from the individual.

Preferably, cortical stem and progenitor cells or cortical neurons produced as described herein for therapeutic applications are clinical grade cells.

A population of cortical stem and progenitor cells or cortical neurons which is administered to an individual may be genetically manipulated to produce a therapeutic molecule, for example a drug or growth factor (Behrstock S et al, Gene Ther 2006 Mar;13(5):379-88, Klein SM et al, Hum Gene Ther 2005 Apr;16(4):509-21)

A pharmaceutical composition, medicament, drug or other composition may comprise a population of cortical stem and progenitor cells or cortical neurons, along with a pharmaceutically acceptable excipient, carrier, buffer, preservative, stabiliser, anti-oxidant or other material well known to those skilled in the art. The precise nature of the carrier or other material will depend on the route of administration. A pharmaceutical composition may be produced by admixing a population of cortical stem and progenitor cells or cortical neurons with a pharmaceutically acceptable excipient, vehicle or carrier, and optionally one or more other ingredients.

The composition may be administered to a patient, e.g. for treatment (which may include preventative treatment) of dysfunctional cortical tissue, as described above.

Liquid pharmaceutical compositions generally include a liquid carrier such as water, petroleum, animal or vegetable oils, mineral oil or synthetic oil. Physiological saline solution, tissue or cell culture media, dextrose or other saccharide solution or glycols such as ethylene glycol, propylene glycol or polyethylene glycol may be included.

The composition may be in the form of a parenterally acceptable aqueous solution, which is pyrogen-free and has suitable pH, isotonicity and stability. Those of relevant skill in the art are well able to prepare suitable solutions using, for example, isotonic vehicles such as Sodium Chloride, Ringer's Injection, or Lactated Ringer's Injection. A composition may be prepared using artificial cerebrospinal fluid.

Cells may be implanted into a patient by any technique known in the art (e.g. Lindvall, O. (1998) Mov. Disord. 13, Suppl. 1:83-7; Freed, C.R., et al., (1997) Cell Transplant, 6, 201-202; Kordower, et al., (1995) New England Journal of Medicine, 332, 1118-1124; Freed, C.R., (1992) New England Journal of Medicine, 327, 1549-1555, Le Blanc et al, Lancet 2004 May 1;363(9419):1439-41).

Administration of a composition is preferably in a "prophylactically effective amount" or a "therapeutically effective amount" (as the case may be, although prophylaxis may be considered therapy), this being sufficient to show benefit to the individual. The actual amount administered, and rate and time-course of administration, will depend on the nature and severity of what is being treated. Prescription of treatment, e.g. decisions on dosage etc, is within the responsibility of general practitioners and other medical doctors.

A composition may be administered alone or in combination with other treatments, either simultaneously or sequentially dependent upon the condition to be treated.

The neurons, cortical stem and progenitor cells or populations thereof may be derived from disease-specific iPS cells, for example DS-iPS cells.

The neurons or stem and progenitors or populations thereof may display neurodegenerative disease pathology, such as intra- or extra-cellular protein aggregation or increased apoptosis. For example, neurons or stem and progenitors derived from DS-iPS cells may display AD pathology.

Cells which display neurodegenerative disease pathology may be useful in screening for active compounds which may be useful in the development of therapeutics.

Cerebral cortex neurons produced as described above may be used in methods of screening for compounds with therapeutic activity, which may be useful in treatment of diseases, such as neurological diseases.

A method of screening for a compound useful in the treatment of a neurological disease may comprise, contacting isolated cerebral cortex neurons produced by a method described above, with a test compound and.
determining the effect of the test compound on said neurons.

For example, the effect of a test compound on neuronal cell death/survival, growth, proliferation, condition, aggregation, electrical activity, synaptic activity and/or gene expression may be determined. A method of screening for a compound useful in the treatment of a neurodegenerative disease may comprise,
determining the effect of a neurotoxin on isolated cerebral cortex neurons produced by a method described above in the presence and absence of a test compound.

A test compound which reduces or ameliorates the effect of the neurotoxin on the neurons may be useful in the treatment of a neurodegenerative disease or the development of therapeutics.

Neurotoxins may include aggregation prone proteins associated with neurodegenerative disease, such as AB42.

Cerebral cortex neurons produced as described herein may also be useful for example in toxicity testing.

A method of determining the neurotoxicity of a compound may comprise,
contacting the compound with isolated cerebral cortex neurons produced by a method described above, and,
determining the effect of the test compound on said neurons.

A compound which alters, for example increases or decreases neuronal cell death/survival, growth, proliferation, condition, aggregation, electrical activity, synaptic activity and/or gene expression may be identified as a neurotoxin.

Preferably, the neurons or stem and progenitors are derived from disease-specific iPS cells, for example iPS cells derived from cells obtained from an individual suffering from a disease associated with damaged or dysfunctional cortical neurons, such as sporadic and familial Alzheimer's disease, familial and sporadic epilepsy, autism, schizophrenia and cerebral palsy.

Neurons or stem and progenitors derived from disease-specific iPS cells may display neurological disease pathology, such as aberrant synaptic or electrical activity, intra- or extra-cellular protein aggregation, aberrant gene expression, or increased cell death.

The effect of the test compound on the neurological disease pathology may be determined. A compound which reduces or inhibits the pathology may be identified as potentially useful in the treatment of a neurological disease or the development of therapeutics against the neurological disease.

Neurological pathologies, such as Alzheimer's disease pathologies, may be measured within 1, 2, 3, 4, 5, 6 or more weeks of initiating differentiation. These pathologies may be measured over 1, 2, 3, 4, 5, 6 or more weeks to determine the effect of the test compound.

A method of screening for a compound useful in the treatment of Alzheimer's disease may comprise,
contacting isolated cerebral cortex neurons produced from DS-iPS cells by a method described above, with a test compound and,
determining the effect of the test compound on said neurons.

For example, the effect of the test compound on one or more Alzheimer's disease pathologies, such as expression levels of Aβ42, the ratio of Aβ42 to Aβ40 (non-toxic form); Aβ42 levels within neurons; Aβ42 levels in the extracellular medium; Aβ42 oligomer formation; levels of hyperphosphorylated Tau; intracellular calcium levels; formation of intra- and extracellular amyloid plaques; and rates of programmed cell death, may be determined.

A decrease in any of these pathologies, relative to control cells not treated with the test compound, may be indicative that the test compound displays an anti-AD activity and may be useful in the treatment of Alzheimer's disease and/or the development of AD therapeutics.

Alzheimer's disease pathologies such as the ratio of Aβ42 to Aβ40 (non-toxic form); Aβ42 levels within neurons; Aβ42 levels in the extracellular medium; Aβ42 oligomer formation; levels of hyperphosphorylated Tau; intracellular calcium levels; expression of Aβ42, formation of intra- and extracellular amyloid plaques; and/or rates of programmed cell death may be measured using standard techniques. For example, the development of amyloid plaques may be determined by live staining with the Thioflavin T analog, BTA1²⁴.

Methods as described herein may comprise the step of identifying a test compound which reduces or ameliorates one or more neurological disease pathologies in the cortical neurons. Compounds which reduce neurological disease pathologies are candidate compounds for treatment of the neurological disease or for the design of such compounds.

Following identification of a compound which reduces or ameliorates one or more neurological disease pathologies in the cortical neurons, a method may further comprise modifying the compound to optimise its pharmaceutical properties. This may be done by modelling techniques as described above.

A test compound identified using one or more initial screens as having ability to reduce or ameliorate one or more neurological disease pathologies in the cortical neurons, may be assessed further using one or more secondary screens.

A secondary screen may involve testing for a biological function or activity *in vitro* and/or *in vivo,* e.g. in an animal model. For example, the ability of a test compound to reduce or ameliorate one or more symptoms or pathologies associated with the neurological disease in an animal model of the disease may be determined.

Following identification of a test compound which reduces or ameliorates one or more neurological disease pathologies in the cortical neurons, the compound may be isolated and/or purified or alternatively it may be synthesised using conventional techniques of recombinant expression or chemical synthesis. Furthermore, it may be manufactured and/or used in preparation, i.e. manufacture or formulation, of a composition such as a medicament, pharmaceutical composition or drug. These may be administered to individuals for the treatment of a neurological disease.

Various further aspects and embodiments of the present invention will be apparent to those skilled in the art in view of the present disclosure.

The cell markers cited herein are all well-known in the art and full details are readily available on public databases, such as the online NCBI database. Antibodies for detecting expression of these markers may be produced by routine techniques or obtained from commercial sources (e.g. Abcam Ltd, Cambridge UK).

"and/or" where used herein is to be taken as specific disclosure of each of the two specified features or components with or without the other. For example "A and/or B" is to be taken as specific disclosure of each of (i) A, (ii) B and (iii) A and B, just as if each is set out individually herein.

Unless context dictates otherwise, the descriptions and definitions of the features set out above are not limited to any particular aspect or embodiment of the invention and apply equally to all aspects and embodiments which are described.

Certain aspects and embodiments of the invention will now be illustrated by way of example and with reference to the figures and tables described below.
Figure 1 shows a quantitative RT-PCR for the ventricular zone stem and progenitor cell-expressed transcription factor Foxg1, which demonstrates that the induction of cortical stem cells begins after 5 days and peaks after 20 days, whereas Tbr2-expressing cells begin to appear almost a week later. Error bars, s.e.m.

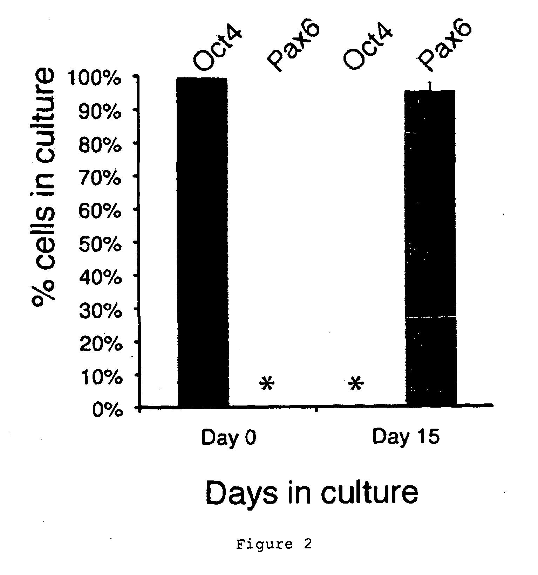

Figure 2 shows the differentiation of Oct4-expressing hES cells at high efficiency to Pax6-expressing neural stem cells over the 15 day neural induction period. Asterisks indicate the absence of detectable Pax6-expressing cells at day 0 and of Oct4-expressing cells at day 15. Error bar, s.e.m., n=3 samples for Pax6-expressing cells at day 15.
Figure 3 shows a quantification of the efficiency of cortical induction, as assayed by the percentage of Pax6-expressing cells (percentage of nuclei, detected with DAPI), in the presence or absence of retinoids in two hESC and four hiPSC cell lines. Values are the average of three cultures for each cell line. Error bars, s.e.m.
Figure 4 shows quantification of the proportions of Tbr2+ Ki67+ cells found in cortical rosettes - between 15 and 20% of cells within rosettes derived from different hESC and hiPSC lines express Tbr2+. Of the Tbr2+ population, approximately 40% are Ki67+ cycling progenitor cells.
Figure 5 shows quantification of the proportions of Tbr2+ cells found in cortical rosettes - between 15 and 20% of cells within rosettes derived from different hESC and hiPSC lines express Tbr2.
Figure 6 shows a time course of neuronal differentiation from hESCs in culture: by day 35, approximately 70% of the cells in the culture are Tuj1-expressing neurons. Counts at each timepoint from n=3 cultures. Error bars, s.e.m.

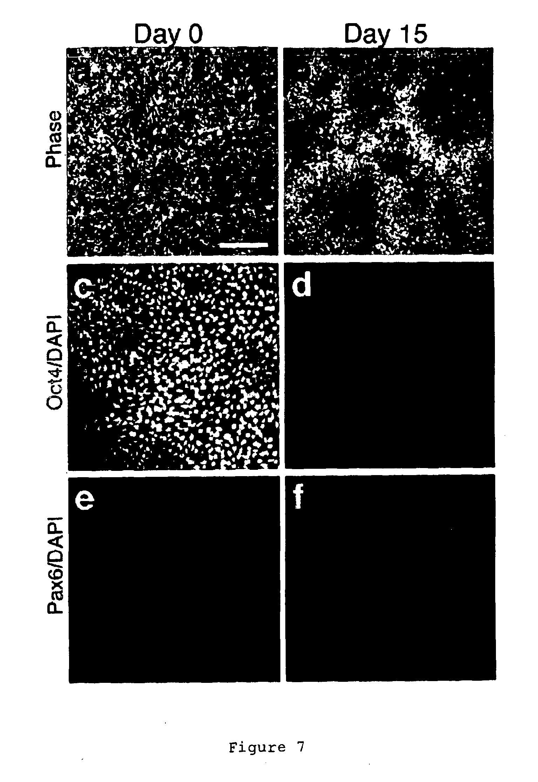

Figure 7 shows the differentiation of Oct4-expressing hES cells (A, C, E) to Pax6-expressing cortical stem and progenitor cells (B, D, F) over a 15-day interval. Scale bars, 100 µm.
Figure 8 shows hES-derived cortical stem and progenitor cells form polarized neuroepithelial rosettes of proliferating cells (Ki67) in which many mitoses (phospho-histone H3) take place near a central lumen (asterisk in i; white arrow in j indicates apical mitosis) formed from the apical surfaces of the neuroepithelial cells (CD133, j). Abventricular mitoses are also commonly found (yellow arrow in j)
Figure 9 shows a subset of the proliferating, Ki67-positive cells within the rosette express the SVZ-specific transcription factor Tbr2 (white arrows, k). However, the majority of Tbr2-expressing cells are newly born, Doublecortin (Dcx)-expressing neurons (white arrows, 1). Scale bars j, m, 100 µm; k, 1, 50 µm.
Figure 10 shows the order of cortical neurogenesis from human ES cells recapitulates normal development. Deep and upper layer neurons are generated in a temporal order from hESCs, with layer 5 and 6 neurons (Tbr1 and CTIP2) generated before layer 2/3 neurons (Brn2). N=3 cultures scored for each marker. Error bars, s.e.m.
Figure 11a shows a diagram of classes of cortical projection neurons in the layers of the adult cortex, based on mouse data, with transcription factors expressed in each class of neuron as indicated. CPNs, callosal projection neurons.
Figure 11b shows the differentiation of early and later born cortical neurons from hESCs. Corticothalamic projection neurons of layer 6 (Tbr1/CTIP2-expressing neurons, g, i) and corticospinal motor neurons of layer 5 (CTIP2-positive, Tbr1-negative neurons, h, white arrows in i) are both present. j) to k) show the differentiation of upper layer, later born cortical neurons from hESCs. These neurons express Cux1, Satb2 and Brn2. Scale bars g-1, 50 µm.
Figure 12 shows the relative proportions of different classes of cortical projection neurons generated from humans ES and iPS lines. Approximately equal proportions of deep and upper layer neurons are generated from all lines.
Figures 13 to 17 show the generation of functional human cortical excitatory neurons from hESCs *in vitro* recapitulates *in vivo* development.

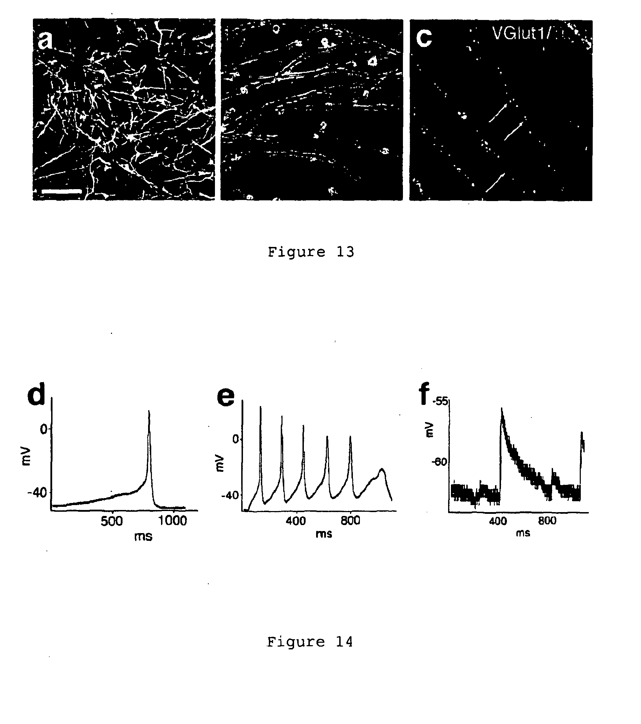

Figure 13 shows the efficient generation of large numbers of neurons (Tuj1-expressing, a) with abundant neurites (b) from hES cells. Almost all neurons are glutamatergic, as evidenced by the presence of the vesicular glutamate transporter in cell bodies and neurites (white arrows, c). Scale bars, 50 µm (a), 25 µm (b), 10 µm (c).
Figure 14 shows human ES-derived cortical neurons mature *in vitro* to fire spontaneous action potentials (d, n=3 neurons). Mature cortical neurons sustain runs of action potentials on current injection, as observed for hES-derived cortical neurons in culture (e, n=21 neurons). These neurons also form functional synapses, demonstrated by the presence of frequent mEPSPs (f, n=12 neurons).

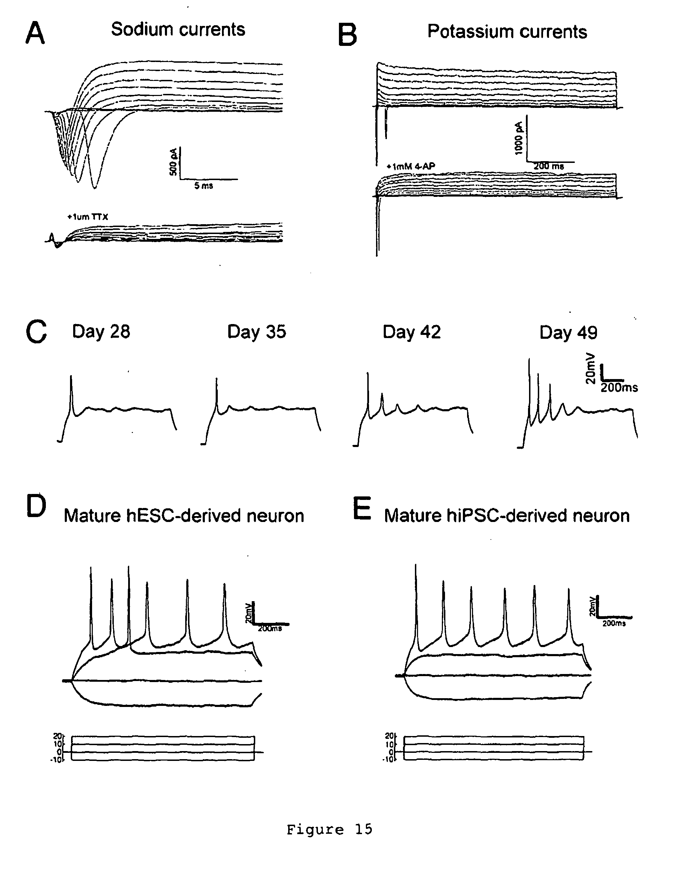

Figure 15 shows that pluripotent stem cell-derived cortical neurons show differentiate to acquire mature electrophysiological properties. Figures 15A and 15B show voltage-gated sodium and potassium channels in PSC-derived cortical neurons. Current responses to families of step depolarizations from a holding potential of -80 mV to +40 are superimposed. In 15A, fast-activating and inactivating inward sodium currents are completely blocked by applying TTX. In 15B, 4-AP blocks a fast-activating transient fraction of outward K current.
Figure 15C shows the electrophysiological properties of PSC-derived cortical neurons mature over time, as exemplified by the change in action potential firing in response to step current injections.
Figures 15D and 15E show hESC and hiPSC-derived cortical neurons develop robust regular-spiking behaviour in response to step current injection.

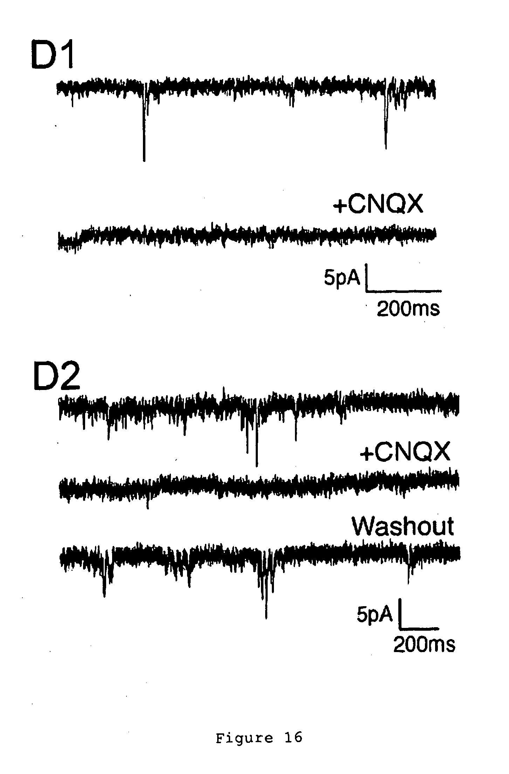

Figure 16 shows the detection of mEPSCs in whole cell recordings of hESC (D1) or hiPSC (D2)-derived cortical neurons. In each case, the AMPA receptor antagonist CNQX blocked the appearance of mEPSCs.

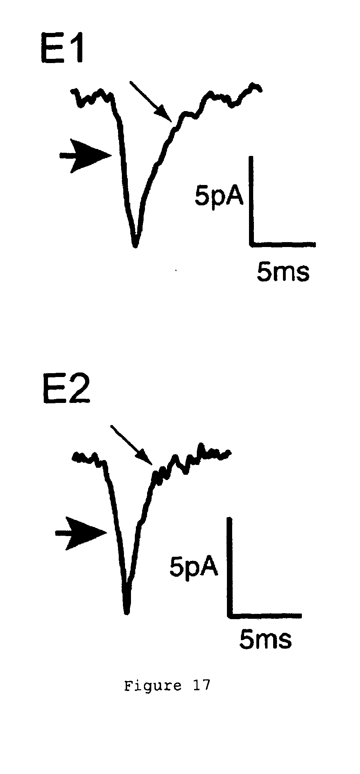

Figure 17 shows the average mEPSC from hESC (n=20 events; E1) and hiPSC (n=20 events; E2) derived cortical neurons. In each case the mEPSC has the characteristic rapid onset (arrowhead) and slow decay (arrow) of AMPA-mediated currents.

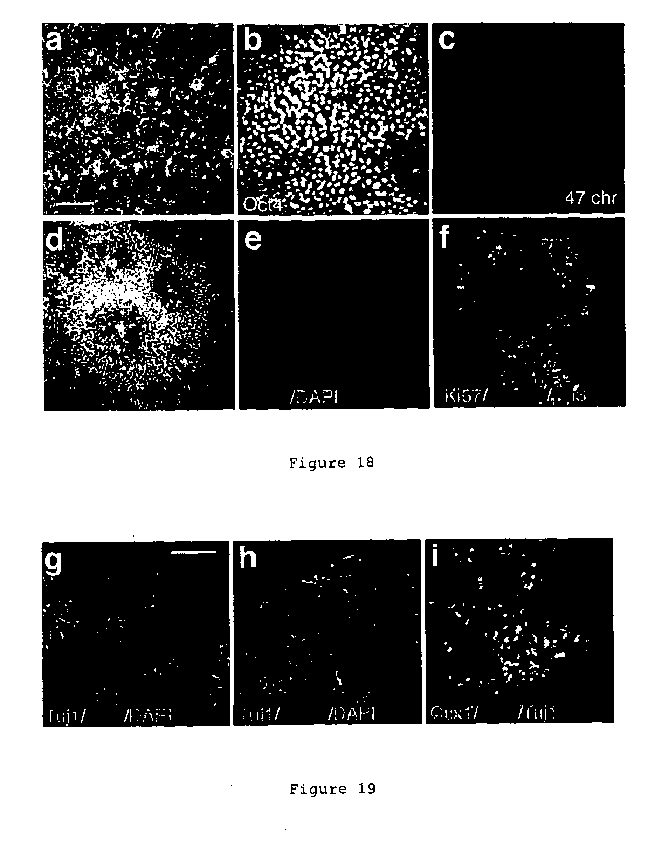

Figures 18 to 31 show the directed differentiation of Down syndrome iPS cells to functional cortical projection neurons
Figure 18 shows the differentiation of Oct4-expressing DS-iPS cells (A-C) to Pax6-expressing cortical stem and progenitor cells in polarized neuroepithelial rosettes (D-F) over a 15-day interval. Karyotyping confirmed that the DS-iPS cells contain 47 chromosomes (C). Scale bars, 50 µm (a-e), 100 µm (f).
Figure 19 shows that DS-iPS generate neurons of all cortical layers: deep layers (Tbr1, g) and upper layers (Cux1, Brn2, Satb2, h, i).

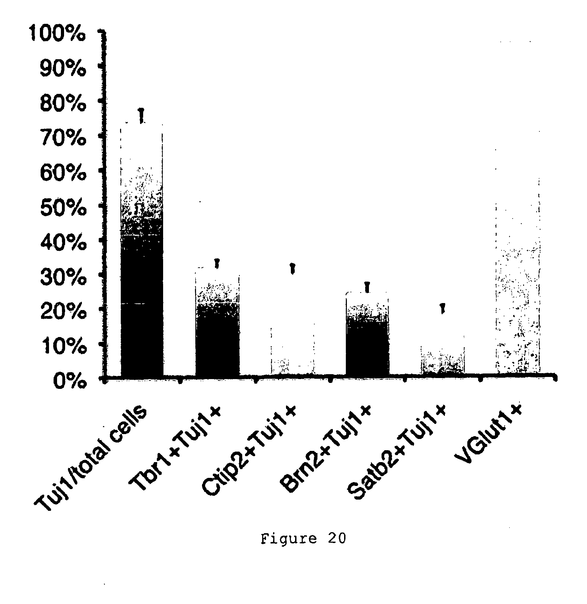

Figure 20 shows that approximately equal numbers of deep and upper layer neurons are generated (j). For each cell specific marker, n=3 cultures; error bars, s.e.m. Scale bars, 50 µm.

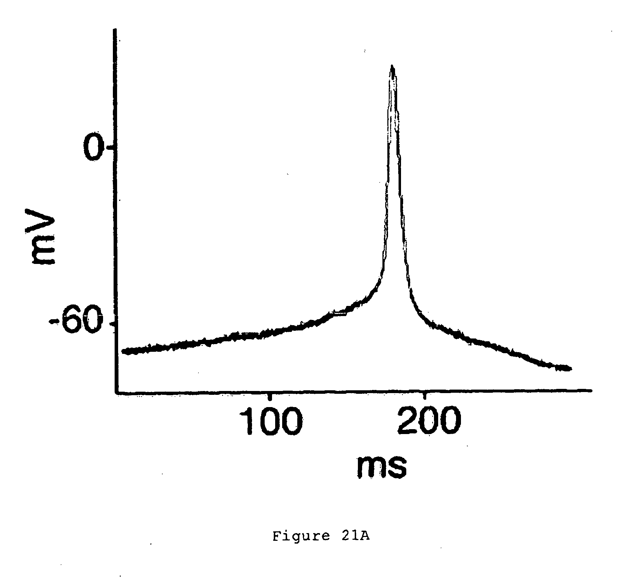

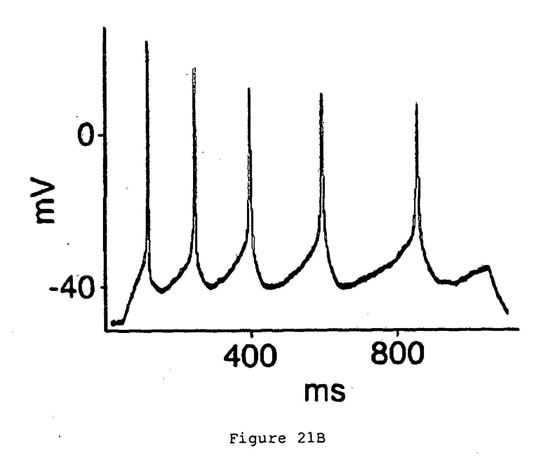

Figure 21 shows DS-iPS-derived cortical neurons become functionally mature *in vitro,* firing spontaneous action potentials (A) and firing trains of action potentials upon current injection (B).

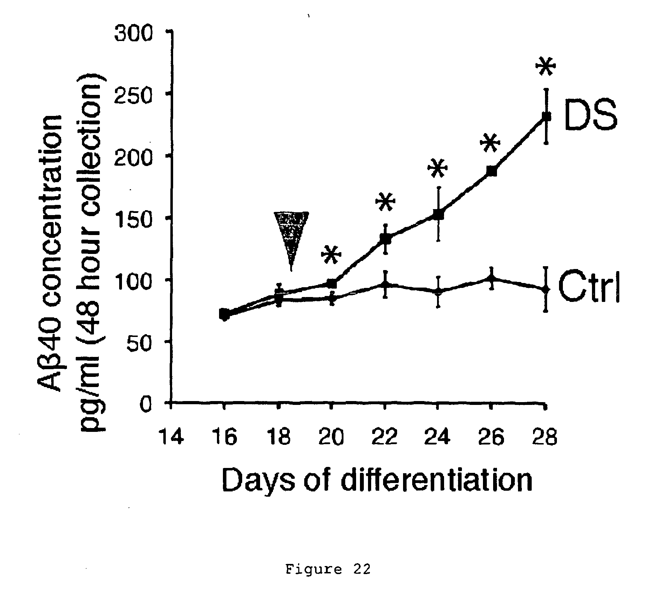

Figure 22 shows the secretion of Aβ40 peptides by control healthy (Ctrl) and DS (DS) iPS cell-derived cortical neurons (n=3 cultures for each cell line). Cell culture media were collected every 48 hours to measure Aβ40 peptide concentrations by sandwich ELISA. Cell culture media were completely refreshed every 48 hours, therefore Aβ40 levels reflect secretion and accumulation over a 48 hour period. The green arrowhead indicates the onset of overt neuronal differentiation in these cultures. Asterisks indicate significant (p<0.025) differences in Aβ40 concentrations between control and DS neurons within a time point.

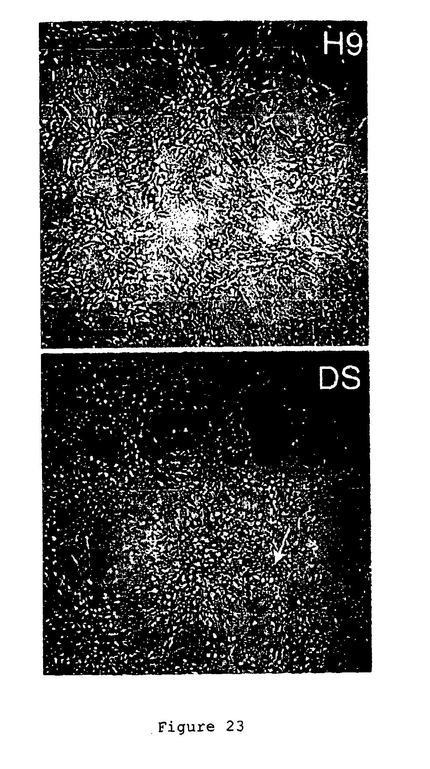

Figure 23 shows the live staining of amyloid in hES and DS-iPS-derived cortical neurons after 90 days in culture, using the Thioflavin analogue BTA-1. BTA-1-positive aggregates are specifically observed in the DS-iPS neuronal cultures (arrowheads in b). H9: H9 hES cell-derived cortical neurons; DS, DS1-iPS4-derived cortical neurons. Scale bar, 100 µm.

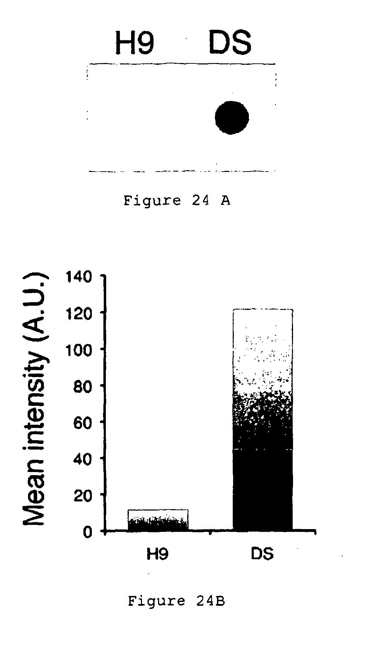

Figure 24 shows dot-blot (a) and quantification (b) for Aβ42 accumulation in cell culture supernatants over 48 hours in day 60 cultures of control (H9) and Down syndrome (DS) cortical neurons.

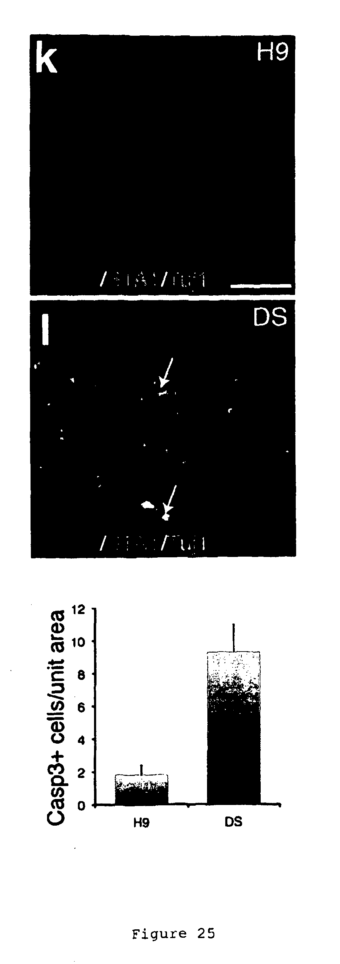

Figure 25 shows large amounts of cell death (activated caspase 3) are observed in DS-iPS cortical neuron cultures, much of which is found in neurons with intracellular BTA1-positive amyloid aggregates (arrows in 1). Quantification of apoptotic cells finds a significantly higher incidence in DS-derived cortical neurons (n=3 cultures of each cell type). Error bars, s.e.m. Scale bar, 50 µm.

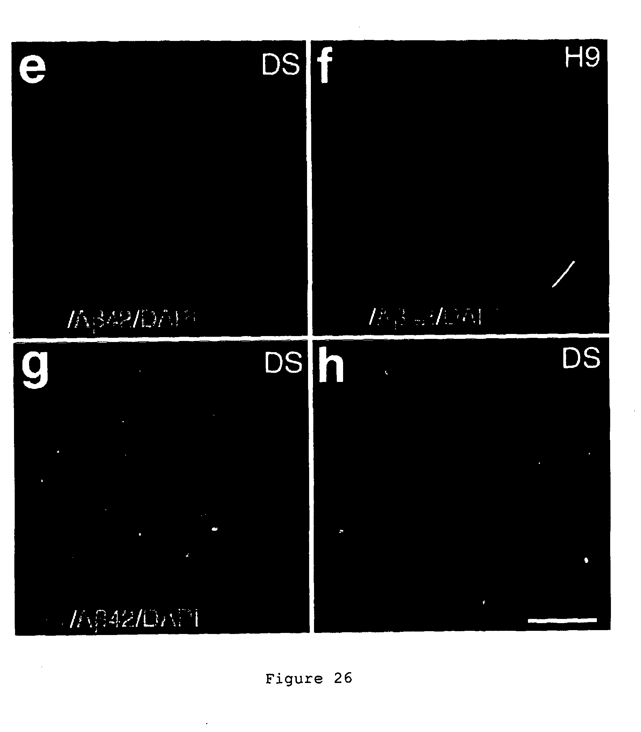

Figure 26 shows the pathogenic Aβ42 fragment of APP is not found in cortical stem cell cultures from DS-iPS cells (e) and very rarely in cultured cortical neurons from hESCs (f) but that large numbers of intracellular and extracellular deposits of Aβ42 are found in cultures of DS-iPS cell-derived cortical neurons between 60 and 90 days of culture. Scale bars e-g, 100 µm; h, 50 µm.

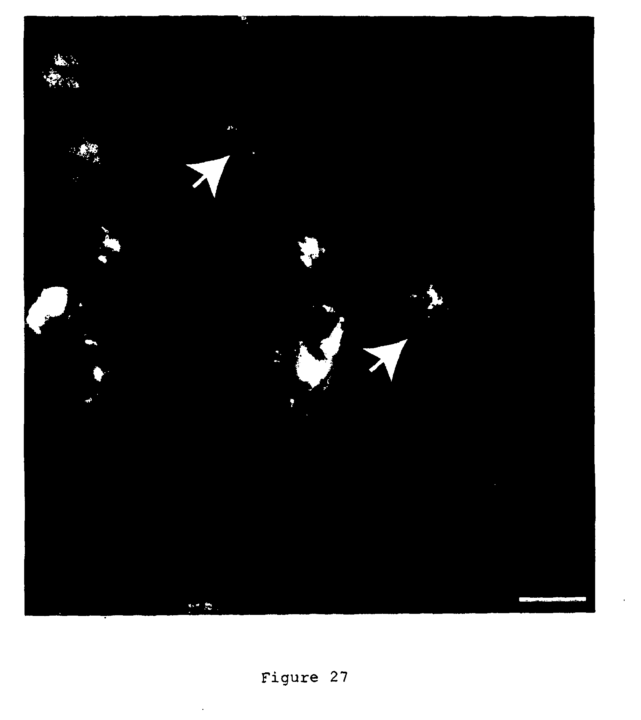

Figure 27 shows that extracellular amyloid plaques (arrowheads) are found around cortical neurites in a 3D rendering of a Z-stack of 90-day cultures of DS-iPS cortical neurons. Scale bar, 25µm.

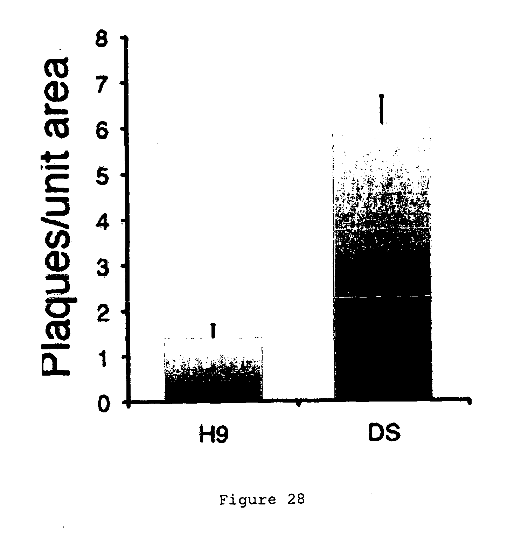

Figure 28 shows quantification of amyloid aggregates in cortical neuron cultures from H9 and DS cells. Aggregates larger than the average diameter of a neuronal cell body were counted in three cultures of each cell type. Error bars, s.e.m.

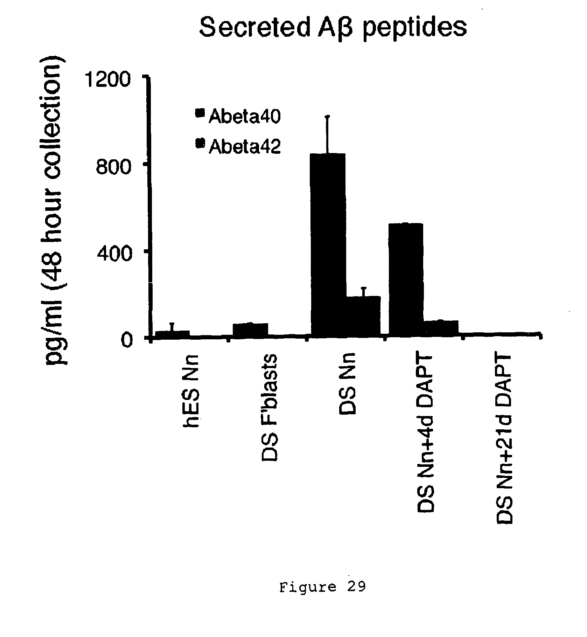

Figure 29 shows DS cortical neurons produce large amounts of soluble extracellular Aβ40 and Aβ42 peptides by day 70 of differentiation, in contrast to DS fibroblasts and age-matched cultures of hES-derived cortical neurons. The secreted Aβ40:Aβ42 ratio is 4.6:1. Inhibition of gamma-secretase with DAPT for 4 days reduces production of both Aβ40 and Aβ42, altering the Aβ40:Aβ42 ratio to 7.5:1. Longer term, 21 day gamma secretase inhibition reduces production of both Aβ peptides to undetectable levels.

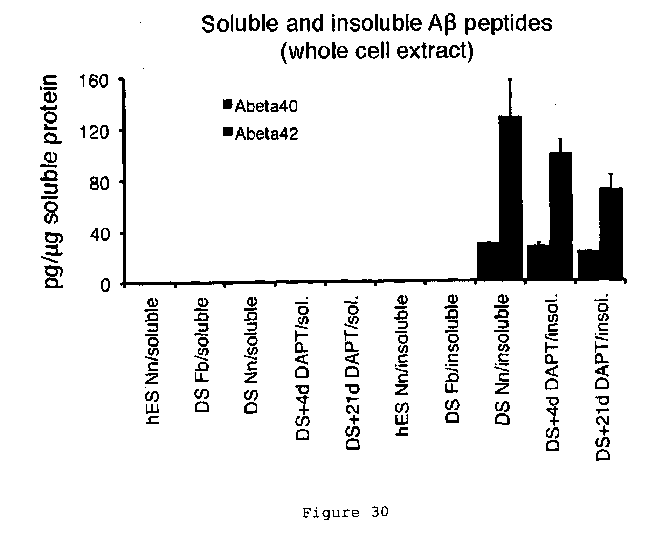

Figure 30 shows quantification of insoluble and insoluble Aβ peptide from whole cell extracts of cultures of DS fibroblasts, hES-derived cortical neurons and DS cortical neurons demonstrates that the only detectable Aβ is in the insoluble fraction and is primarily Aβ42. Gamma-secretase inhibition does not significantly alter the amounts of insoluble Aβ42 present in the cultures.

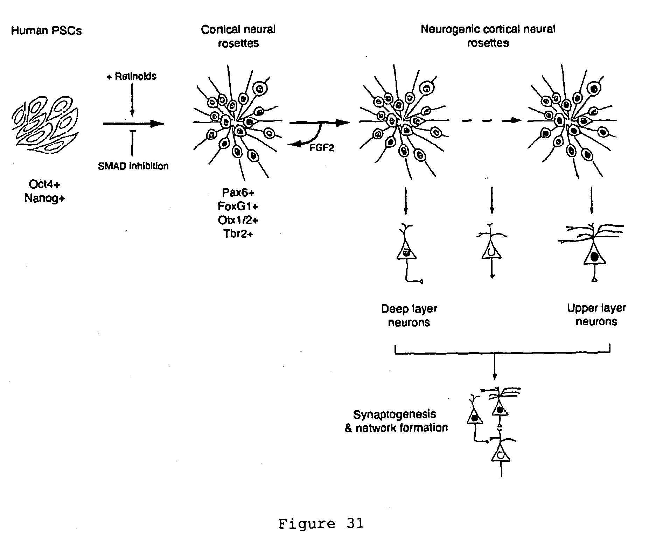

Figure 31 shows a schematic of the differentiation procedure described herein: a combination of dual SMAD inhibition, combined with retinoids, differentiates PSCs to cortical stem and progenitor cells that can be expanded/maintained with FGF2. Removal of FGF2 allows neurogenesis to take place, with cortical stem cells following the same developmental progression in the genesis of cortical cell types as occurs in vivo. Cortical projections of all layers are generated from PSCs and form networks of functional excitatory, glutamatergic synapses.

### Experiments (Reference)

### Methods

### Human pluripotent stem cell culture

Human ES cell research was approved by the Steering Committee for the UK Stem Cell Bank and for the Use of Stem Cell Lines, and carried out in accordance with the UK Code of Practice for the Use of Human Stem Cell Lines. Culture of hES (H9, WiCell Research Institute, and Edi2, kind gift from Jenny Nichols, Cambridge Centre for Stem Cell Research) and hiPS cell lines (DS1-iPS4, Harvard Stem Cell Institute²²) was carried out on mitomycin-treated mouse embryonic fibroblasts (MEFs) according to standard methods 13. Briefly, cells were maintained in hESC medium (all components Invitrogen unless otherwise stated): DMEM/F12 containing 20% KSR, 6 ng/ml FGF2 (PeproTech), 1mM L-Gln, 100 µm non-essential amino acids, 100 µM 2-mercaptoethanol, 50 U/ml Penicillin and 50 mg/ml Streptomycin.

### Directed differentiation to cerebral cortex

hESCs or iPSCs were isolated from MEFs following dissociation to single cells with Accutase (Innovative Cell Technologies) by a 1 hr pre-plate on gelatin-coated dishes in hESC medium supplemented with 10 ng/ml FGF2 and 10 µM ROCK inhibitor (Calbiochem). The non-adherent pluripotent stem cells were harvested and plated on Matrigel (BD) coated 12-well plates in MEF-conditioned hESC medium with 10

ng/ml FGF2. Once the cell culture reached 95% confluence, neural induction was initiated by changing the culture medium to a culture medium that supports neural induction, neurogenesis and neuronal differentiation (referred to as 3N medium), a 1:1 mixture of N2- and B27-containing media. N2 medium: DMEM/F12, N2 (GIBCO), 5 µg/ml insulin, 1mM L-glutamine, 100 µm non-essential amino acids, 100 µM 2-mercaptoethanol, 50 U/ml Penicillin and 50 mg/ml Streptomycin; B27 medium: Neurobasal (Invitrogen), B27 with vitamin A (GIBCO), 200 mM Glutamine, 50 U/ml Penicillin and 50 mg/ml Streptomycin. 3N medium was supplemented with 500 ng/ml mouse Noggin-CF chimera (R&D Systems) and 10 µm SB431542 (Tocris) to inhibit TGFβ signalling during neural induction. Cells were maintained in this medium for 8-11 days, during which time the efficiency of neural induction was monitored by the appearance of cells with characteristic neuroepithelial cell morphology. Neuroepithelial cells were harvested by dissociation with Dispase and replated in 3N medium including 20 ng/ml FGF2 on poly-ornithine and laminin-coated plastic plates. After a further 2 days, FGF2 was withdrawn to promote differentiation. Cultures were passaged once more with Accutase, replated at 50,000 cells/cm2 on poly-ornithine and laminin-coated plastic plates in 3N medium and maintained for up to 90 days with a medium change every other day.

### Immunocytochemistry and quantification

Cultures were fixed in 4% paraformaldehyde in PBS and processed for immunoflourescent staining and confocal microscopy. Antibodies used for this study: Tbr1 (Abcam), Tbr2 (Millipore), CTIP2 (Abcam), Prominin/CD133 (Abcam), phosphorylated Histone H3 (Abcam), Pax6 (Chemicon), Oct4 (Abcam), Ki67 (BD), Doublecortin (Santa Cruz), β-Tubulin III (Chemicon), β-Tubulin III (Covance), vGlut1 (Synaptic Systems), Cux1 (Santa Cruz), Brn2 (Santa Cruz), Satb2 (Abcam), cleaved Caspase 3 (Cell Signaling), C-terminus of Aβ42 (Chemicon). Secondary antibodies used for primary antibody detection were species-specific, Alexa-dye conjugates (Invitrogen).

For quantifying numbers of cells expressing specific cortical neuronal markers, cell cultures were dissociated into single cells with Accutase and resuspended at a density of 100,000 cells/ml. 20,000 cells were plated onto each poly-lysine coated glass slide with a Cytospin Centrifuge (Thermo Scientific), and fixed and stained for confocal microscopy. For live staining of amyloid in neuronal cultures, BTA-1 (Sigma) in DMSO was added to a final concentration of 100 nM for 20 minutes before washing and imaging. Statistical comparisons of cell counts were carried out using Student's t-test. Superresolution microscopy imaging of synaptic proteins was carried out using standard fixation and staining techniques, visualized with a Deltavision OMX system (Applied Precision).

Quantification of extracellular Aβ42 was carried out by dot-blotting of 10 µl of cell culture supernatant from day 65 cultures of DS and H9 cortical neurons.

Supernatants were filtered to remove cellular debris (0.22 µm filter) before blotting. Aβ42 was detected with a polyclonal antibody specific to the C-terminus of Aβ42 (Chemicon). Dot-bots were quantified in ImageJ (NIH).

### Aβ peptide detection and quantification

Quantification of secreted Aβ40 and Aβ42 was carried out by sandwich ELISA (Invitrogen) of 50 µl of cell culture supernatant from cultures of DS, iPS control and H9 hES cortical neurons. For quantification of water-soluble and insoluble Aβ peptides, water- and formic acid-soluble protein fractions from whole cell extracts of cortical cultures were prepared and levels of each peptide measured by ELISA (Invitrogen). Inhibition of gamma-secretase was carried out in DS cortical cultures by addition of DAPT (Calbiochem) every 48 hours from day 50 of differentiation onwards.

### Quantitative RT-PCR

Total RNA was isolated from three cultures at each timepoint (days 5, 10, 15, 20 and 25) in both ES and DS-iPS cultures (Trizol, Sigma). Total RNA was reverse transcribed and used for quantitative RT-PCR with primers specific to Foxg1 and Tbr2 using the Applied Biosystems 7000 system.

### Electrophysiology

Whole cell current clamp recordings were performed at room temperature in artificial cerebral spinal fluid containing (in mM): 125 NaCl, 25 NaHCO3, 1.25 NaH2PO4, 3 KCl, 2 CaCl2, 1 MgCl2, 25 Glucose, 3 Pyruvic Acid, bubbled with 95% 02, 5% CO2. Borosilicate glass electrodes (resistance 6-10 MΩ) were filled with an intracellular solution containing (in mM): 135 K-Gluconate, 7 NaCl, 10 Hepes, 2 Na2ATP, 0.3 Na2GTP, 2 MgCl2. Cells were viewed using a BW50WI microscope (Olympus) with infrared DIC optics. Recordings were made with a Multiclamp 700A amplifier (Molecular Devices). Signals were filtered at 4kHz, sampled at 20kHz with 16-bit resolution, and analysed using Matlab(MathWorks).

### Slice culture of human cortical neurons

Coronal slices of embryonic mouse brain (embryonic day 16) were prepared as 250 µm thick sections using a Leica VT1000S Vibratome. Brain slices were cultured on permeable membrane inserts in Costar Transwell plates, to which N2B27 (3N) medium was added below the membrane. Early stage (day 35 of differentiation) human ESC and iPSC-derived cortical neurons were dissociated to single cells and plated onto the mouse brain slices, essentially as described23. Cultures were maintained for 14 days, before fixing and processing for immunostaining with antibodies to Tbr1 (recognized both mouse and human) and human-specific NCAM antibodies.

### Results

We explored a number of different approaches by which we could first direct differentiation of hES cells to neural stem and progenitor cells of the cerebral cortex in monolayer culture, using defined media and known signalling pathways.

Cortical stem/progenitor cells were identified by their expression of the transcription factors Foxg1, Pax6, Otx1/2 and Tbr2, with no expression of genes normally expressed in ventral telencephalon or more caudally (Nkx2.1, D1x1 and HoxB4). The final demonstration of cortical induction rested on the neuronal output from the neural stem/progenitor cells differentiated from human ES and iPS cells, as discussed below.

Retinoids regulate the transition from neural stem cell expansion to neurogenesis in the mouse cerebral cortex and augment the derivation of glutamatergic neurons from mouse ES cells. In the absence of retinoids, neural induction from hES cells was found to be highly inefficient.

We found that combining retinoid signalling with inhibition of TGFβ signalling promoted neural induction directed differentiation of hESCs to cerebral cortex stem and progenitor cells. Using this approach, almost all cells (>95%) in the culture are Pax6 - expressing neural stem cells fourteen days after the initiation of neural induction.

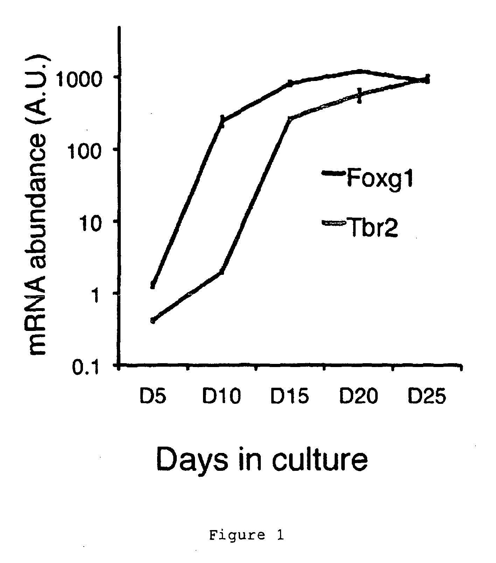

Neural stem cell cultures derived by this approach express high levels of Foxg1 mRNA and subsequently express Tbr2 mRNA (Fig. 1). We tested whether the dependency of cortical differentiation from human embryonic stem cells on retinoids generalized to other pluripotent stem cell lines, both ES and iPS.

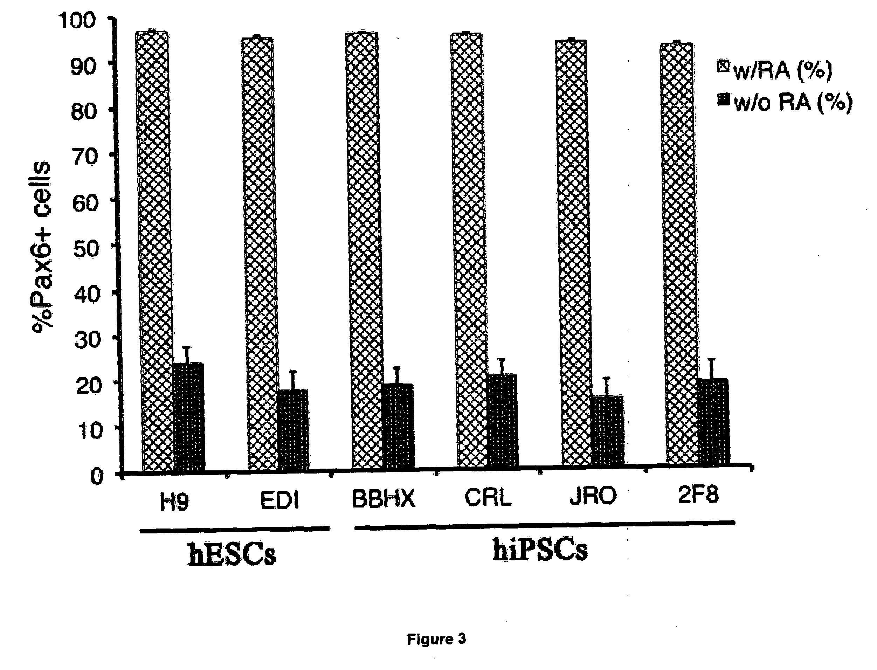

We differentiated two human ESC lines and four iPSC lines to neural stem cells using the method described here in the presence or absence of retinoids. The two hESC lines were derived in two different institutions (H9 and Edi2), and the four iPSC lines were derived by two different groups. In the absence of retinoids, cortical neural induction from hES and hiPS cells by dual SMAD inhibition was inefficient, as assessed by Pax6 expression (Fig. 3). Patches of Pax6-expressing cells were observed, accounting for approximately 25% of the cells in each culture (Fig. 3), but the majority of cells were Pax6-negative. In contrast, inclusion of retinoids (retinol acetate and all-trans retinol) resulted in robust, efficient differentiation of all lines to cortical neural stem/progenitor cells, with almost all cells expressing Pax6 (Fig. 3) and Otx1/2. This was the case for all hES and hiPS, independent of origin or derivation method.

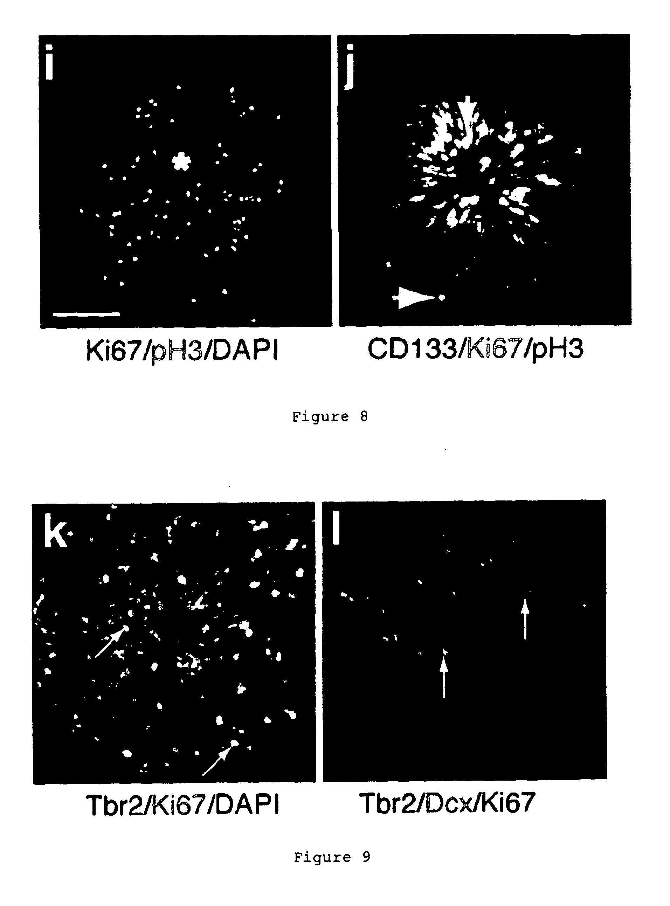

In addition to the expression of transcription factor combinations unique to cortical stem and progenitor cells (Pax6, Foxg1, Otx1/2 and Vimentin), the cortical neural vrosette cells reported here display features which are characteristic of the cortical neuroepithelium in vivo. Human ESC-derived neural stem cells form rosette structures following neural induction (Rovelet-Lecrux, A., et al. Nat Genet 38, 24-26 (2006); Quon, D., et al. Nature 352, 239-241 (1991)). The cortical rosettes have obvious apico-basal polarity, localizing CD133/prominin, the transferrin receptor and aPKC to the apical (luminal) extreme of each cell (Fig. 8j). Furthermore, many mitoses are located at the apical/luminal surface of each rosette, as occurs in vivo. Finally, as occurs in vivo, they localize centrosomes (visualized by ASPM and CEP135 protein localization) to the extreme apical end of each cell, typically extending into the central lumen of each rosette (Fig. 8j). This is consistent with the presence of both apical and basal cortical stem and progenitor cells within the cultures. The majority of the mitotic cells displaced from the lumen of the neuroepithelial rosettes express Pax6.

A signature feature of neuroepithelia is the process of interkinetic nuclear migration (IKNM), during which the location of the nucleus of each stem/progenitor cell moves during the cell cycle: the nuclei of G1 cells start at the apical surface and migrate towards the basal surface, undergoing S-phase away from the ventricular/apical surface, before undergoing directed nuclear translocation during G2 and mitosis at the apical surface. The localization of the majority of M-phase nuclei to the centre of each rosette, at the apical surface, provided indication that IKNM takes place in rosettes. To confirm this, we used time-lapse imaging of cell movements in cortical rosettes observe nuclear movements and mitoses.

Consistent with the phospho-histone H3 staining, the majority of mitoses took place at the apical/luminal surface, with a smaller number occurring towards the periphery of the rosette. All apical mitoses were preceded by a G2-phase in which the nucleus moved from an abventricular position, typically several nuclear diameters away from the lumen of the rosette. This G2-phase, apically directed movement typically took place over a period of several hours. The presence of IKNM, together with the polarization of the rosettes and the presence of both apical and basal mitoses, indicate that cortical rosettes mimic the linear in vivo cortical neuroepithelium by forming circular epithelial discs.

In the developing cerebral cortex in vivo, the main population of cortical stem cells forms a polarized, pseudostratified neuroepithelium, whereas secondary populations of progenitor cells are found within the inner and outer subventricular zones, referred to as the SVZ and oSVZ, respectively (Hansen, D.V et al. Nature 464, 554-561 (2010); Fietz et al Nat Neurosci 13, 690-9 (2010)). Neural stem cells derived by the methods reported herein contain at least two and possibly three populations of stem and progenitor cells: the majority of cells within the rosettes, which are Pax6+/Otx+/Ki67+, apico-basally polarized cells with radial processes, and which undergo IKNM and apical mitoses; a second population of cells that undergo abventricular or basal mitoses; and a third Tbr2+/Ki67+ population. The presence of a small population of Tbr2-positive proliferative cells (Fig. 8i) is consistent with the outer subventricular zone (OSVZ) progenitor cells.

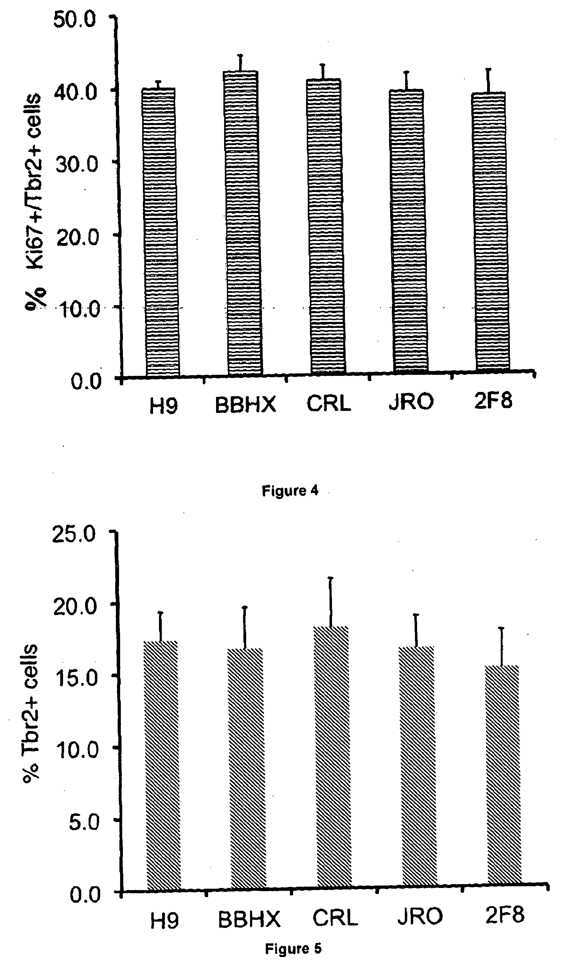

Whereas approximately half of Tbr2-expressing cells are Ki67-expressing, cycling progenitor cells (Fig. 4), the other half are newly born, Doublecortin-positive neurons as previously described in the developing human cortex in vivo (Hansen et al (2010) supra). The Tbr2/Ki67+ population makes up an average of 15% of the cells in each rosette day 25 (Fig. 5), and contributes substantially to the neuronal output from the stem/progenitor cell populations.

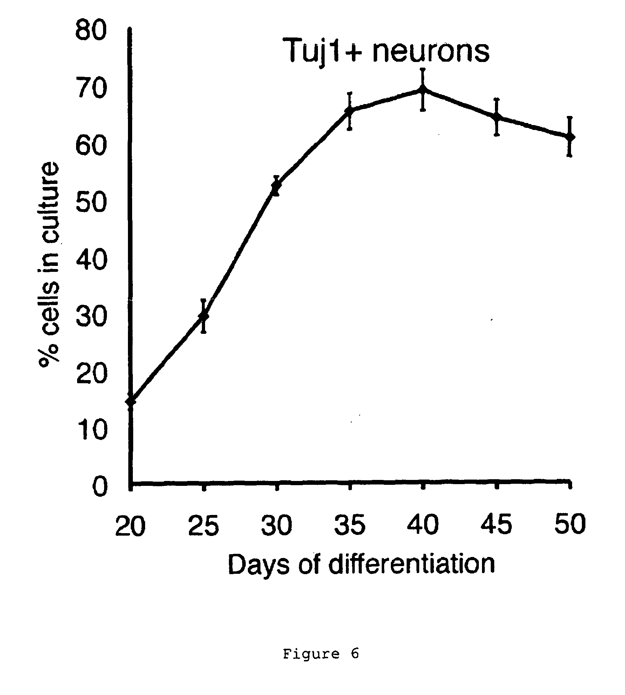

Cortical neurogenesis, stimulated by the withdrawal of FGF2 from the culture medium, takes place for over two months following neural induction from hES and hiPS cells (Fig. 6). This is consistent with the approximately 70 day period of cortical neurogenesis in humans (Caviness et al supra) compared with the six day cortical neurogenetic period in mice (Takahasi et al J Neurosci 16, 6183-96 (1996)).

PSC-derived cortical stem and progenitor cells generate exclusively glutamatergic projection neurons, and no detectable GABAergic interneurons. The initial wave of neurogenesis includes deep, Tbr1-expressing layer projection neurons, confirming the cortical identity of the rosettes. Rosettes begin to generate astrocytes relatively late in the process, with astrocytes appearing around day 70. Although GABAergic interneurons are not generated under cortical induction conditions, at early stages cortical rosettes are plastic with respect to regional identity: treatment with the hedgehog signalling agonist, purapomorphine, ventralises the rosettes, resulting in the genesis of GAD67+ GABAergic interneurons.

To further assess the differentiation of PSC-derived cortical neurons, we used the cortical slice culture assay (Polleux et al Sci STKE 2002, PL9 (2002)) to analyse their ability to migrate, terminally differentiate and orient within the mouse cerebral cortex. Dissociated PSC-derived neurons were plated on coronal slices of developing mouse brain (embryonic day 14.5) and cultured for 14 days. Human PSC-derived cortical neurons survived in large numbers within the mouse cortex and extended abundant neurites. Strikingly, the majority of human neurons oriented radially within the developing mouse cortex, orthogonal to the ventricular surface, with a subset aligning tangentially in the marginal zone. Finally, human PSC-derived neurons maintained their layer identity when cultured in mouse cortex, with many, for example, expressing the layer 6 transcription factor Tbr1.

Key features of cortical neurogenesis in all mammals are the multipotency of cortical stem and progenitor cells and their ability to generate excitatory glutamatergic neurons of different laminar fates in a stereotyped temporal order (Mountcastle et al supra). Human corticogenesis from hES cells recapitulates in vivo cortical development: human cortical projection neurons of each cortical layer are generated in the correct temporal order and at high efficiency from hES cells. Astrocytes are also generated at a late stage in this culture system.

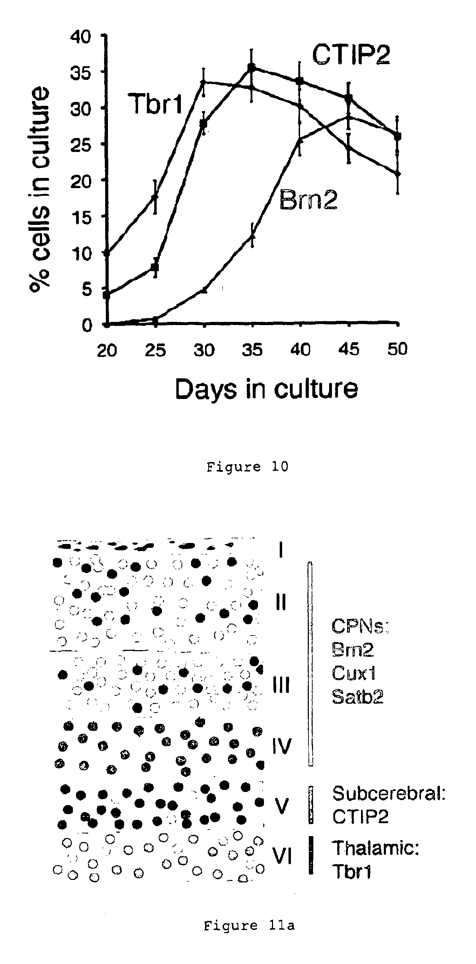

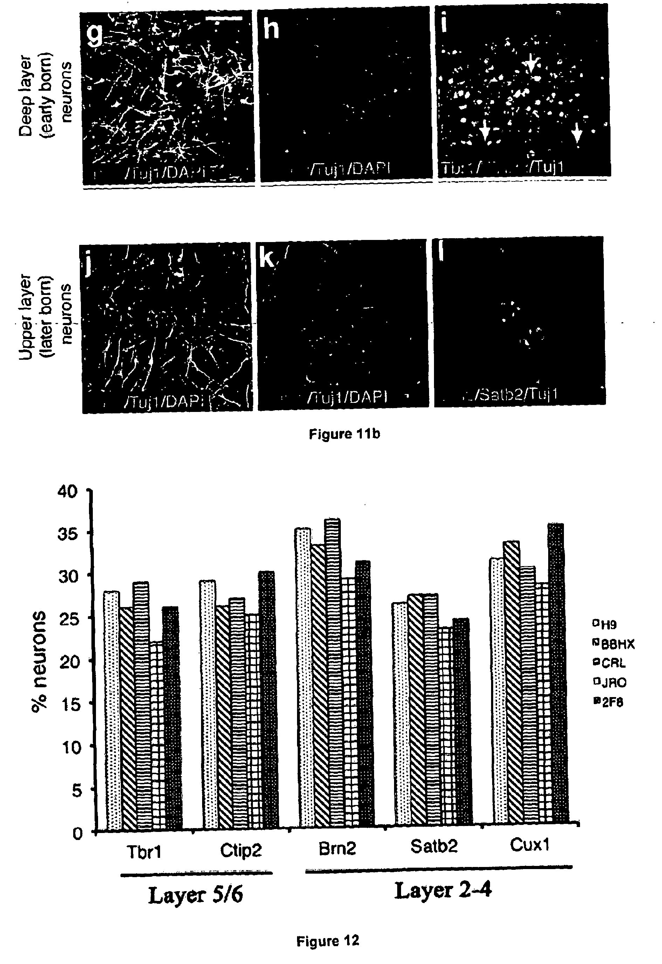

Glutamatergic projection neurons of the adult cortex are generated in a stereotyped temporal order, with deep layer neurons produced first and upper layer neurons last. In rodents, cortical glutamatergic neurons of different laminar fates and projection types can be defined by their expression of different transcription factor combinations (Fig. 11a): Tbr1+/CTIP2- (low or absent) layer 6/corticothalamic projection neurons ; CTIP2+/Tbr1- layer 5/subcortical projection neurons; Cux1+/Brn2+ layer 2-4 neurons; and Satb2+ layer 2-4 callosal projection neurons.

We used the expression of these factors in neurons to study the time course of cortical projection neuron subtype differentiation from PSCs over a 70-day interval, beginning from the withdrawal of FGF2. Deep, layer 6 neurons (Tbr1+) appear first, followed by CTIP2-expressing layer 5 and 6 neurons. Upper layer, Brn2/Cux1 callosal projection (layer 2-4) neurons differentiate subsequently, beginning between days 25-30, with Satb2 expression appearing late, between days 65 and 80 (Figs. 10-12). The same temporal order of projection neuron production was observed from four different hiPSC lines. The combinations of transcriptions factors described above were used to quantify the proportions of different cortical projection neuron types after 70 days in culture. Importantly, roughly equal numbers of deep and superficial layer neurons were present in this system (Figs. 10-12), in contrast with previous reports of reduced production of upper layer neurons from mouse ES cells and the minimal production of upper layer neurons in human ESC-derived aggregate cultures. Again, the proportions of different projection neuron subtypes generated from hESCs and four different hiPS lines was notably similar (Figs. 10-12). Astrocytes were generated at a late stage in this culture system, after neurons of all cortical layers, as occurs in vivo.

Human corticogenesis from hES cells as described herein therefore recapitulates in vivo cortical development: human cortical projection neurons of each cortical layer are generated in the correct temporal order and at high efficiency from a polarized neuroepithelium.

The hES-derived cortical stem and progenitor cells generate exclusively glutamatergic projection neurons, and no detectable interneurons, when cultured in conditions to promote neurogenesis (Fig. 13). After approximately 90 days in culture, functional synapses, as indicated by the presence of miniature excitatory post-synaptic potentials (mEPSPs) and foci of synaptophysin immunofluorescence were detectable in hES-derived cortical neuron cultures (Fig. 14). These neurons therefore spontaneously fire action potentials and develop mature firing properties over several weeks *in vitro*.

We confirmed that PSC-derived cortical projection neurons differentiate as functional neurons (Fig. 15A, B). Whole-cell patch-clamp recordings from individual cells (total n=54 neurons) demonstrated the presence of voltage-gated sodium currents, blocked by tetrodotoxin (TTX), and voltage-gated potassium currents, a transient
component of which was blocked by 4-aminopyridine (4-AP). Thus, PSC-derived cortical neurons, from both hESCs and hiPSCs, terminally differentiate appropriately to excitable cells.

Cortical projection neurons terminally differentiate over days-weeks in the neonatal rodent,cortex to develop mature firing properties. A similar process takes place in primary cultures of rodent cortical neurons. As hESC- and hiPSC-derived cortical neurons age *in vitro,* their ability to fire bursts of action potentials in response to current injection increased with time (Fig. 15C): young (day 28) neurons typically fired a single action potential, whereas older (day 49) neurons fired up to 5 action potentials following current injection. By day 65, many neurons (n=32) robustly fired trains of action potentials when stimulated with steps of current injection. This maturation process was observed in both hES and hiPS-derived cortical neurons (Fig. 15D, E), and is similar to the maturation process that occurs *in vivo.*

Synaptogenesis is the critical step in neural network formation. The formation of physical synapses among PSC-derived cortical projection neurons was detected using super resolution (structured illumination) microscopy to visualize pre- and post-synaptic protein localization. Synapses were defined as regions in the 100s of nanometers in diameter found on dendrites (detected by MAP2 staining) where proteins specific to the pre- and post-synaptic compartments were juxtaposed. Two different pairs of pre- and post-synaptic proteins were used to identify synapses: PSD95, enriched at the excitatory, glutamatergic postsynaptic density, together with synaptophysin, a major synaptic vesicle protein; and Homer1, a widely-expressed postsynaptic density protein, with the presynaptic protein Munc13-1. Foci of PSD95 in the 100nm size range were abundant on the surface of dendrites of neurons generated from both hESCs and hiPSCs. Juxtaposed, but non-overlapping, ~100nm foci of presynaptic (Synaptophysin, Munc13-1) and post-synaptic (PSD95, Homer1) proteins were also abundant in cultures of both hESC and hiPSC-derived cortical neurons as early as day 28. Finally, to test whether the observed physical synapses are functional, we used whole cell patch-clamp recording to detect miniature excitatory post-synaptic currents (mEPSCs), in cultures between days 45 and 100 (total n=48 neurons). In cultures generated from all hESC and hiPSC lines, mEPSCs 5-10 pA in amplitude were frequently detected (Fig. 16). The mEPSCs were blocked by the AMPA receptor blocker, CNQX (Fig. 16), and had the characteristic kinetics of AMPA receptor-mediated synaptic currents, with rapid onset and a late, slow decay (Fig 17). We conclude that human PSC-derived cortical neurons form networks of functional glutamatergic synapses in culture.

To address the potential of this method for generating patient-specific cortical neurons and modelling neurological diseases, such as Alzheimer's disease, we used this approach to direct differentiation of disease-specific human induced pluripotent stem cells (iPS) to cortical neurons. Down syndrome/Trisomy 21 is the commonest genetic cause of mental retardation, occurring in approximately 1/700-800 live births (Wiseman, F.K et al Hum Mol Genet 18, R75-83 (2009)). Individuals with Down syndrome have a very high incidence of Alzheimer's disease, attributed to the presence of the amyloid precursor protein (APP) gene on chromosome 21(Selkoe, D.J.Biol Chem 271, 18295-18298 (1996)). Duplication of the APP gene in humans results in autosomal dominant early-onset dementia, and mice with increased APP gene dosage develop amyloid plaques and the neuropathological hallmarks of Alzheimer-type dementia. We applied the process described here to differentiate human healthy control and human Down syndrome iPS cells (DS1-1PS422) to cortical neurons (Figs. 18 to 21). Control and Down syndrome hiPS (referred to here as DS-iPS) cells generate cortical stem and progenitor cells at high efficiency, again developing as polarized neuroepithelial rosettes (Fig. 18). Cortical neurons of each layer differentiate in the correct temporal order and on the same timescale from DS-iPS cells as from hES cells (Figs. 19 and 20). As for the hES-derived cortical neurons, these neurons are glutamatergic and electrically active (Fig. 21).

A key step in the development of Alzheimer's disease in vivo is the increased generation of short, 38-42 amino acid Aβ peptides from APP by glutamatergic neurons in the cerebral cortex (Mountcastle 1998 supra) a process that can occur in people with Down syndrome in their teens and twenties (Rovelet-Lecrux, A., et al. Nat Genet 38, 24-26 (2006).

We monitored the secretion of Aβ40 and Aβ42 peptides by cortical neurons derived from control and DS iPS cells (Fig.22). Neurons typically produce considerably more Aβ40 than Aβ42. However, Aβ42 is considered to be the primary pathogenic Aβ peptide in Alzheimer's disease, as it self-aggregates to form soluble and insoluble oligomers and insoluble fibrils and plaques that can also contain Aβ40. Secretion of the Aβ40 peptide initially becomes detectable at low levels for both control and DS cortical neurons within days of the onset of neuronal differentiation (Fig. 22). However, DS cortical neurons exponentially increase their production of Aβ40 to high levels over the subsequent 10 days, whereas control neuron production of Aβ40 remains consistently low (Fig. 22). Secretion of the pathogenic Aβ42 peptide by control or DS cortical neurons was not detected at this early stage of neuronal differentiation.

Given the increasing generation of Aβ40 by DS cortical neurons over time, we assayed the development of amyloid plaques in DS-iPS derived cortical neurons in culture by live staining of intracellular and extracellular aggregates of amyloid with the Thioflavin T analog, BTA1²⁴ (Fig. 23) . Over a three-month period after the initiation of cortical differentiation, DS-iPS cortical neurons generate both intracellular and extracellular aggregates of BTA1-labelled amyloid. In contrast, BTA1-positive neurons or extracellular aggregates were rarely observed in cultures of hES-derived cortical neurons over the same period.

Aβ42 peptides form soluble and insoluble oligomers that inhibit synaptic function in the early stages of Alzheimer's disease. The Aβ42 peptide is produced at very high levels by DS-iPS cortical neurons and accumulates in the cell culture medium (Fig.24).

We found that many of the DS-iPS neurons with detectable intracellular amyloid aggregates undergo programmed cell death, as assayed by activated caspase-3 staining (Fig. 25).

Immunofluorescence and confocal microscopy demonstrated the presence of numerous Aβ42-containing aggregates both within and outside DS-iPS cortical neurons, with extracellular Aβ42-positive aggregates often around neurites (Figs 26 and 27), indicating that Aβ42 production increases over time from DS cortical neurons. In contrast, Aβ42 is produced at much lower levels (approximately 10-fold lower) and Aβ42-containing aggregates are much less frequently found in cultures of hES derived cortical neurons (Fig. 28). The increased production of Aβ42 and the formation of plaques in Alzheimer's disease are associated with neuronal cell death in the cerebral cortex.

Extraction and analysis of the secreted, soluble intracellular and insoluble protein fractions from older, day 70, cultures of DS and control hES cortical neurons confirmed that older DS neurons secrete considerable amounts of both Aβ40 and Aβ42 in a 48 hour period, whereas hES cortical neurons generate a small amount of Aβ40 and no detectable Aβ42 (Fig. 29). Notably, the ratio of Aβ40 to Aβ42 was 4.6:1 in DS cortical neuron cultures, reflecting that DS cortical neurons do not simply generate more Aβ peptides but also change the relative amounts of Aβ40 and Aβ42 produced, as also occurs in sporadic Alzheimer's disease in vivo. High-level secretion of Aβ peptides was specific to DS neurons, as production of Aβ peptides by DS fibroblasts was barely detectable (Fig. 29). In vivo, Aβ42 peptides form soluble and insoluble oligomers that inhibit synaptic function in the early stages of Alzheimer's disease (Palop et al Nat Neurosci 13 812-818 (2010)). In addition to the ELISA detection of secreted, soluble Aβ40 and Aβ42 peptides, soluble Aβ oligomers were also present in the cell culture medium of DS cortical neurons.

In contrast with secreted, soluble Aβ peptides, soluble intracellular Aβ peptide levels were below the level of detection in both DS and control hES-derived cortical neurons (Fig. 30). However, insoluble Aβ40 and Aβ42 were both found in DS cortical neuron cultures, with none found in healthy cortical neuron cultures (Fig. 30). The majority of insoluble Aβ in DS cultures was Aβ42, with Aβ40 4-fold less abundant (Fig. 30), the reverse of the ratio seen for secreted Aβ. Finally, we asked whether Aβ40 and Aβ42 peptide generation and secretion by DS-iPS cortical neurons could be reduced by inhibition of the gamma-secretase complex, one of the two essential protease complexes that process APP to generate Aβ peptides. Short-term, four day, inhibition of gamma-secretase reduced Aβ40 and Aβ42 production by almost half, while longer term treatment (21 days) reduced secretion of both Aβ peptides to below detectable levels (Fig. 29). However, inhibition of gamma-secretase did not significantly reduce the amount of insoluble Aβ42 in DS cortical neuron cultures (Fig. 30), which is likely to be due to the absence of clearance mechanisms for removing amyloid in cortical neuron cultures.

In conclusion, we have developed a process by which human pluripotent stem cells can be directed to differentiate to cerebral cortex neurons, recapitulating *in vivo* development. This process consists of a number of distinct steps: the directed differentiation of human PSCs to form a complex population of cortical stem and progenitor cells; an extended period of cortical neurogenesis; a late phase of astrocyte genesis; neuronal terminal differentiation to acquire mature electrophysiological properties; and synaptogenesis and network formation. In contrast to previous reports of directed differentiation of mouse ESCs, the diversity of cortical projection is reproduced in this system, with approximately equal proportions of both deep (early born) and upper (late born) layer neurons generated over several months in culture.

The critical step in this process is the highly efficient differentiation of human PSCs to cortical neural stem/progenitor cell rosettes, and the genesis of both apical and basal progenitor cells in this system. We have found that inhibition of sonic hedgehog does not promote cortical induction, and retinoids are required for efficient cortical induction from human PSCs under the neural induction conditions used here. This finding is consistent with the regulation by retinoids of the transition from neural stem cell expansion to neurogenesis in the mouse cerebral cortex and the retinoid dependency of the derivation of glutamatergic neurons from mouse ES cells.

Primary mouse cortical stem/progenitor cells generate projection neurons when grown at clonal density in vitro, although they produce many fewer upper layer/late born neurons than in vivo. Withdrawal of mitogenic FGF2, which can be used to expand rosette cells, promotes the onset of neurogenesis in this system. The temporal order of genesis of projection neurons of each layer is preserved in this system: deep, layer 6 neurons are the first to appear, whereas layer 2/3 neurons are the last neurons generated, followed by astrocytes.

In contrast to mouse, where cortical neurogenesis takes place over a six-day interval, human cortical neurogenesis continues for approximately 100 days in vivo. We find that the period of genesis of projection neurons in this system approximates to that seen in the human embryo: deep layer 6 neurons are generated within days of the onset of neurogenesis, whereas upper layer, Satb2-expressing corticofugal neurons do not begin to appear until approximately 60 days later.

The ability to generate all classes of cortical projection neurons by directed differentiation of PSCs is likely to be due to the presence of both apical and basal progenitor cell types within cortical rosettes. We observe at least two and possibly three distinct populations after cortical induction: the majority of cells within the rosettes are Pax6+/Otx+/Ki67+, apico-basally polarized cells with radial processes, which undergo IKNM and apical mitoses; a second population of stem/progenitor cells that undergo basal mitoses; and a Tbr2+/Ki67+ population. These populations approximate to three populations of stem/progenitor cells in the developing human cortex: VZ neuroepithelial cells, SVZ cells and oSVZ cells. The populations of stem/progenitor cells generated in this system are competent to reliably generate the diversity of projection neuron types found in the human cortex in vivo, providing indication that the complexity of human cortical progenitor types is captured by this system.

The ability to model cortical development from cortical induction through to excitatory synapse and network formation will enable future functional studies of human cortical development and can be exploited to produce specific cortical cell types, such as corticospinal motor neurons. As this approach is equally efficient from human ESCs and iPSCs, patient-specific cortical neurons can be generated for disease modelling, and potentially for therapeutic purposes

We have demonstrated the potential of this system for modelling diseases of the cerebral cortex and adult-onset neurological disease by studying the development of early-onset Alzheimer's disease in Down syndrome iPS cell-derived cortical neurons. The generation of Aβ42 and the formation of amyloid plaques occur in this system over a period of months, compared to typically several decades in vivo. The use of DS-iPS-derived neurons to model the pathology of early-onset Alzheimer's disease described here provides a potentially powerful *in vitro* system for analyzing normal and pathological functions of APP and its peptide products in cortical synaptic function and neurodegeneration.

## Claims

1. A method for the *in vitro* induction of corticogenesis of human pluripotent cells comprising;
(i) providing a population of isolated human pluripotent stem cells,
(ii) culturing the population under culture conditions which stimulate retinoid signalling and inhibit TGFβ and BMP signalling,
such that said population differentiate into cortical stem and progenitor cells.

2. A method according to claim 1 wherein the population are cultured in a neural induction medium which comprises a retinoid, and one or more TGFβ-SMAD signalling inhibitors.

3. A method according to claim 2 wherein the retinoid is retinoic acid, all-trans retinol or retinol acetate.

4. A method according to claim 2 or claim 3 wherein the one or more TGFβ-SMAD signalling inhibitors are selected from 4-[4-(1,3-benzodioxol-5-yl)-5-(2-pyridinyl)-1H-imidazol -2-yl] benzamide, noggin and dorsomorphin.

5. A method according to claim 4 wherein the neural induction medium comprises 4-[4-(1, 3-benzodioxol-5-yl)-5-(2-pyridinyl)-1H-imidazol -2-yl] benzamide and noggin and optionally insulin.

6. A method according to any one of claims 1 to 5 wherein the population of human pluripotent stem cells is cultured for up to 20 days.

7. A method according to any one of the preceding claims comprising expanding and/or storing the population of cortical stem and progenitor cells.

8. A method according to any one of the preceding claims comprising allowing the population of cortical stem and progenitor cells to differentiate into cerebral cortex neurons.

9. A method according to any one of the preceding claims wherein the human pluripotent stem cells are iPS cells.

10. A method according to claim 9 wherein the iPS cells are derived from a sample of healthy cells obtained from an individual with a damaged or dysfunctional cerebral cortex.

11. A method according to claim 10 wherein the iPS cells are derived from a sample of cells with a disease associated phenotype or genotype.

12. A method according to claim 11 wherein the iPS cells are Down syndrome iPS cells (DS-iPS) derived from a sample of Down syndrome cells and optionally, the method comprises detecting or measuring one or more Alzheimer's disease pathologies or age-related pathologies in the cerebral cortex neurons produced from said DS-iPS cells.

## Patentansprüche

1. Verfahren zur *In*-*vitro*-Induktion von Corticogenese menschlicher pluripotenter Zellen, das Folgendes umfasst:
(i) das Bereitstellen einer Population von isolierten, menschlichen pluripotenten Stammzellen,
(ii) das Kultivieren der Population unter Kulturbedingungen, die Retinoidsignalisierung stimulieren und TGFβ- und BMP-Signalisierung hemmen,
so dass die Population zu kortikalen Stamm- und Vorläuferzellen differenziert.

2. Verfahren nach Anspruch 1, worin die Population in einem neuralen Induktionsmedium kultiviert wird, das ein Retinoid und einen oder mehrere TGFβ-SMAD-Signalisierungsinhibitoren umfasst.

3. Verfahren nach Anspruch 2, worin das Retinoid Retinsäure, all-trans-Retinol oder Retinolacetat ist.

4. Verfahren nach Anspruch 2 oder Anspruch 3, worin der eine oder die mehreren TGFβ-SMAD-Signalisierungsinhibitoren aus 4-[4-(1,3-Benzodioxol-5-yl)-5-(2-pyridinyl)-1H-imidazol-2-yl]benzamid, Noggin und Dorsomophin ausgewählt sind.

5. Verfahren nach Anspruch 4, worin das neurale Induktionsmedium 4-[4-(1,3-Benzodioxol-5-yl)-5-(2-pyridinyl)-1 H-imidazol-2-yl]benzamid und Noggin und gegebenenfalls Insulin umfasst.

6. Verfahren nach einem der Ansprüche 1 bis 5, worin die Population menschlicher pluripotenter Stammzellen bis zu 20 Tage lang kultiviert wird.

7. Verfahren nach einem der vorangegangenen Ansprüche, das das Vermehren und/oder Lagern der Population von kortikalen Stamm- und Vorläuferzellen umfasst.

8. Verfahren nach einem der vorangegangenen Ansprüche, das das Ermöglichen des Differenzierens der kortikalen Stamm- und Vorläuferzellen zu Hirnrindenneuronen umfasst.

9. Verfahren nach einem der vorangegangenen Ansprüche, worin die menschlichen pluripotenten Stammzellen iPS-Zellen sind.

10. Verfahren nach Anspruch 9, worin die iPS-Zellen von einer Probe gesunder Zellen stammen, die von einem Individuum mit einer beschädigten oder dysfunktionalen Hirnrinde erhalten wurden.

11. Verfahren nach Anspruch 10, worin die iPS-Zellen von einer Probe von Zellen mit einem mit einer Krankheit assoziierten Phänotyp oder Genotyp stammen.

12. Verfahren nach Anspruch 11, worin die iPS-Zellen Down-Syndrom-iPS-Zellen (DS-iPS) sind, die von einer Probe von Down-Syndrom-Zellen stammen, und gegebenenfalls das Verfahren das Detektieren oder Messen einer oder mehrer Pathologien der Alzheimer-Krankheit oder altersbedingter Pathologien in den aus den DS-iPS-Zellen produzierten Hirnrindenneuronen umfasst.

## Revendications

1. Procédé d'induction *in vitro* de la corticogenèse de cellules pluripotentes humaines comprenant :
(i) la mise à disposition d'une population de cellules souches pluripotentes humaines isolées,
(ii) la mise en culture de la population dans des conditions de culture qui stimulent la signalisation rétinoïde et inhibent la signalisation du TGFβ et des BMP,
de sorte que ladite population se différencie en cellules souches et progénitrices corticales.

2. Procédé selon la revendication 1, dans lequel la population est mise en culture dans un milieu d'induction neurale qui comprend un rétinoïde, et un ou plusieurs inhibiteurs de la signalisation du TGFβ-SMAD.

3. Procédé selon la revendication 2, dans lequel le rétinoïde est l'acide rétinoïque, le rétinol tout-trans ou l'acétate de rétinol.

4. Procédé selon la revendication 2 ou la revendication 3, dans lequel le un ou plusieurs inhibiteurs de la signalisation du TGFβ-SMAD sont sélectionnés parmi le 4-[4-(1,3-benzodioxol-5-yl)-5-(2-pyridinyl)-1H-imidazol-2-yl]benzamide, la noggine et la dorsomorphine.

5. Procédé selon la revendication 4, dans lequel le milieu d'induction neurale comprend du 4-[4-(1,3-benzodioxol-5-yl)-5-(2-pyridinyl)-1H-imidazol-2-yl]benzamide et de la noggine, et facultativement de l'insuline.

6. Procédé selon l'une quelconque des revendications 1 à 5, dans lequel la population de cellules souches pluripotentes humaines est mise en culture pendant 20 jours maximum.

7. Procédé selon l'une quelconque des revendications précédentes, comprenant l'expansion et/ou le stockage de la population de cellules souches et progénitrices corticales.

8. Procédé selon l'une quelconque des revendications précédentes, comprenant le fait de permettre à la population de cellules souches et progénitrices corticales de se différencier en neurones du cortex cérébral.

9. Procédé selon l'une quelconque des revendications précédentes, dans lequel les cellules souches pluripotentes humaines sont des cellules iPS.

10. Procédé selon la revendication 9, dans lequel les cellules iPS sont dérivées d'un échantillon de cellules saines obtenues chez un individu présentant un cortex cérébral lésé ou dysfonctionnel.

11. Procédé selon la revendication 10, dans lequel les cellules iPS sont dérivées d'un échantillon de cellules présentant un phénotype ou un génotype associé à une maladie.

12. Procédé selon la revendication 11, dans lequel les cellules iPS sont des cellules iPS du syndrome de Down (DS-iPS) dérivées d'un échantillon de cellules du syndrome de Down et, facultativement, le procédé comprend la détection ou la mesure d'une ou de plusieurs pathologies de la maladie d'Alzheimer ou pathologies liées à l'âge dans les neurones du cortex cérébral produits à partir desdites cellules DS-iPS.
